# EUROPEAN PATENT SPECIFICATION

(11) **EP 1 585 487 B1**
(45) Date of publication and mention of the grant of the patent: **15.10.2008**
(21) Application number: 04739057.0
(22) Date of filing: 08.01.2004
(51) Int. Cl.: A61K 8/02, A61K 8/33, A61K 8/34, A61K 8/362, A61K 8/37, A61Q 19/10

(54) **PRODUCTS COMPRISING AN APPLICATOR AND A WAX DISPERSION**
EIN APPLIKATOR UND EINE WACHSDISPERSION ENTHALTENDE PRODUKTE
PRODUITS COMPRENANT UN APPLICATEUR ET UNE DISPERSION DE CIRE

(30) Priority: 08.01.2003 EP 03075102
(43) Date of publication of application: 19.10.2005
(73) Proprietor: JOHNSON & JOHNSON GmbH, 40474 Düsseldorf (DE)
(72) Inventor: HAUSER, Matthias, 53757 St. Augustin (DE); SANS, Anne, 50858 Köln (DE)
(74) Representative: Metten, Karl-Heinz
(86) International application number: PCT/EP2004/000981
(87) International publication number: WO 2005/074864

(56) References cited:
- EP-A- 1 365 060
- WO-A-00/42961
- WO-A-03/071985
- DE-A- 10 327 707
- DE-A- 19 738 623
- FR-A- 2 833 163
- US-A- 5 547 676
- US-A1- 2001 025 021
- US-A1- 2003 041 773
- DATABASE WPI Derwent Publications Ltd., London, GB; AN 2003-834646 XP002294490 & CN 1 153 814 A (HUAYU SCI & TECHNOLOGY IND. CORP.) 9 July 1997 (1997-07-09)

## Description

### Field of the Invention

This invention concerns products for cleansing and other applications, which products comprise an applicator such as a puff (pouf), pad, sponge, cotton ball, swab, brush, glove, mitt or bar, to which a wax dispersion has been applied. The invention further concerns the manufacture and use of such products.

### Background of the Invention

A plurality of applicators for delivering commodities to a surface have been developed, such applicators being of varied nature, in as well presentation as material selection, e.g. applicators that are resilient or non-resilient, or that are re-usable or disposable.

Such applicators have been used to apply to a surface ingredients in the form of creams, pastes, gels, liquids, powders and the like. In particular such applicators have been used to apply topical preparations to the skin such as cosmetic, dermatological and the like products. Applicators have been used with a separate product supply or have been impregnated or coated with a measured quantity of product.

One particular type of applicators are wipes, which have become an important product category that has found a wide variety of applications for adults and babies. Examples include face or body cleansing wipes, wipes for skin treatment and skin conditioning wipes.

Over the last couple of decades so-called 'wet wipes' have become successful as products particularly suited for these applications.. These products typically are manufactured by impregnating wipes made of non-woven fabric with a suitable lotion.

Developments in the wipe area were focused on the wipe itself, as well as on the wipe material and on the lotions applied thereto. Lotions have been developed which offered skincare benefits in addition to the basic cleansing properties of the wipe.

However, these types of products still leave room for improvement. Firstly, only a small portion of the lotion is released from the wipes during use. Thus a large quantity of the relatively expensive lotion is not delivered to the skin providing no benefit to the consumer and is wasted when the product is discarded after use. This also prevents the use of expensive but more effective ingredients. Secondly, from a formulation point there is an apparent contradiction in the optimization of cleansing performance and skincare benefits in one single lotion since ingredients that are effective in cleansing usually are not compatible with efficient skin care agents.

Another important factor in cleansing is the fact that a number of soils are water-compatible and therefore more easily removed by water-based formulations, whereas others are lipid-compatible and therefore adequately removed by lipid or oil based formulations. A complete and effective removal of soils therefore requires the presence in or on a wipe of as well water as oil-based components.

This is in particular the case in products used for babies and infants. Inadequate cleaning not only results in personal discomfort but also gives rise to diaper rash and other infection related phenomena. It has been shown that the most effective way of preventing diaper rash is to cleanse the skin thoroughly and to remove the microorganisms that have been identified as causative. The source of these microorganisms is often the fecal deposits that can remain on a baby's skin while wearing the diaper. Because fecal deposits consist of both water-soluble and oil-soluble matter, however, complete removal of fecal deposits from the diaper area requires both water-based and oil-based cleansing agents.

US-4,987,632 discloses a substantially dry-to-the-touch wiping article for use in cleaning soiled surfaces wherein moisture barriers cover the surface of the sheet. WO 99/13861 and US-6,153,208 disclose substantially dry personal cleansing articles wherein the substrate comprises multiple layers. US-6,280,757 concerns cleansing articles that are dry comprising a substrate having apertures of certain size and frequency.

In WO 99/42961 A2 dispersible cleansing article is disclosed which comprises a) a water insoluble substrate comprising an apertured first layer having a specific water flux rate and a second layer attached to said first layer, and b) a cleansing component comprising a surfactant which is positioned between the first and the second layer. A therapeutic benefit component can be disposed adjacent to said water insoluble substrate. This benefit composition can be slut coated onto the surface of the substrate. With the dispersible cleansing article of WO 00/42961 A2 effective cleansing as well as therapeutic or aesthetic benefits to the skin and hair shall be provided in a convenient, inexpensive and sanitary manner.

WO 03/071985 A2 discloses a system for hair removal which includes a hair removal solution, a first pad for applying the hair removal solution, and a package enclosing the first pad in a first inner pouch. A suitable aqueous hair removal solution is reported to comprise about 8 weight % mineral oil, about 3 weight % calcium hydroxide, about 6 weight % cetearyl alcohol, about 6 weight % ceteareth-20, about 1 weight % collagen (90 % concentration), about 0.001 weight % tocopherol, about 2 weight % calcium thioglycolate, about 2 weight % lanolin and a fragrance.

In Chinese patent application CN 1 153 814 A a cloth detergent is described which is compounded with common synthetic detergents as main material and into which melamine, sodium alginate, alum, paraffin, bees wax, earth wax, sodium bicarbonate and water have to be mixed. This cloth detergent shall be suited to clean various waterproof cloth of chemical fibre, wick, silk, bast fibre, cotton and blend fibres.

DE 103 27 707 A1 is about cosmetical sponges having a maximum water uptake capacity in the range from 0.4 to 3.5 g/cm³ and which can be treated with surfactants, fatty components, water soluble polyols, natural or chemically modified natural polymers, synthetic polymers, alpha-hydroxy carbonic acids, vitamins, plant extracts, anti perspirants, natural or synthetic silicates, pigments, inorganic and organic sun protection filters, cosmetical abrasives, oxidants and reducing agents, anti-inflammatory agents, adstringents or proteins.

In US 5,547,676 a cosmetic product having stabilized redox potential is disclosed which comprises a metal selected from the group consisting of zinc, copper, iron, molybdenum, vanadium, silicium, nickel, manganese and titanium, and a cosmetic composition comprising, in an aqueous medium, a water-soluble salt of said metal. These products shall be suited as anti-wrinkle skin-care creames or as sunscreens. The stability of the redox potential of the cosmetic product of US 5,547,676 enables the properties of the cosmetic composition to be exceptionally well preserved.

According to DE 197 38 623 A1 an aqueous skin cleansing emulsion is disclosed comprising alkyl or alkenyl oligoglycoside carbonates and an oil. Suitable oils comprise Guerbet alcohols, esters of linear C₆₋₂₂ fatty acids, triglycerides based on C₆₋₁₀ fatty acids as well as the vegetable oils, dialkylethers and a silicone oils. Such emulsions shall have a very high cleansing capacity both with respect to waxes, oils and silicon compounds and with respect to pigments.

FR 2 833 163 A1 describes a solid mascara composition which comprises, in a cosmetically acceptable aqueous medium, a wax, a film-building polymer and a wetting agent, chosen among the non-ionic wetting agents or amphoteric agents having a HLB balance larger or equal to 10. The mascara composition has a dry extract content higher or equal to 55% by weight, compared with the total mascara weight.

US 2003/0041773 A1 is about a solvent-free polish composition useful for cleaning and polishing operations which comprises wax, a non-drying oil, water and optionally potentional additives such as abrasives, emulsifiers, thickeners, preservatives, colorants, taste-induced deterrents, fragrances, processing aids and preservatives. The wax shall be added to an oil either pre-heated or then subsequently heated with stirring. Optionally, water and/or an acetic acid source, preferably vinegar, can be added either before or after partly of completely cooling the heated wax/oil mixture.

In US 2001/0025021 A1 an emulsifiable wax is disclosed which comprises co-polymers of from 90 to 95 weight % of ethylene, from 4 to 10 weight % of one or more C₃₋₁₂ alkenecarboxylic acids, and from 0 to 1.2 weight % of one or more tertiary esters of the corresponding C₃₋₁₂ alkene carboxylic acids. The wax has to have a cinematic melt viscosity of from 800 to 3000 mm²/s, measured at 120 °C. These emulsifable waxes shall have a very low speck count, have adequate viscosity and are not tacky.EP 1 365 060 A2 discloses a coating formulation comprising a blend of poly(vinyl acetate) emulsion and a paraffin wax emulsion or a vinyl acetate-ethylene polymer emulsion and a paraffin wax emulsion. Said mixtures shall generate a hydrostatic head barrier when applied as a coating to a substrate sufficient to prevent passage or fluids but allow passage of water vapor through it. Suitable substrates can be selected from a non-woven web, an absorbent pad, a non-woven textile and a textile fabric.

EP 1 365 060 A2 discloses a coating formulation comprising a blend of poly(vinyl acetate) emulsion and a paraffin wax emulsion or a vinyl acetate-ethylene polymer emulsion and a paraffin wax emulsion. Said mixtures shall generate a hydrostatic head barrier when applied as a coating to a substrate sufficient to prevent passage or fluids but allow passage of water vapor through it. Suitable substrates can be selected from a non-woven web, an absorbent pad, a non-woven textile and a textile fabric.

The products according to the present invention, which comprise an applicator that contains a wax dispersion, have been found to be effective cleansing tools and efficient applicators of skincare ingredients.

### Summary of the Invention

This invention relates to products according to the features of claim 1.

The applicators may be made of a variety of materials which are structured such that they are capable of holding and/or absorbing a lipid and an aqueous phase. The materials of which the applicators are made therefore may be porous or absorbent in nature. The materials in particular are polymeric and may be both from natural and non-natural origin.

Preferably, the wax dispersion is present at the surface or at the surface portion of one or several sides of the applicator.

The present invention is claims concerned with products that comprise an applicator, other than a porous or absorbent sheet, whereto a wax dispersion has been applied, wherein the wax dispersion comprises :
(a) a wax phase comprising suitable wax components, said wax phase having a melting point or melting range which is above ambient temperature, or which in particular is equal or higher than 25 °C, and
(b) an aqueous phase.

The wax phase mentioned under (a) is present in an amount of 1 - 75 w/w-% and the aqueous phase is present in an amount of 25 - 99 w/w-%, relative to the total weight of the dispersion. In further specific embodiments, the wax dispersion further contains a suitable emulsifier.

The present invention is concerned with products that comprise an applicator whereto a wax dispersion has been applied wherein the wax dispersion comprises wax particles containing components selected from dialkyl(ene) ethers, dialkyl(ene) carbonates, dicarbonic acids and hydroxyfatty alcohols, and any mixture of these components.

Further in particular, the present invention is concerned with products, as defined above, wherein the wax particles have an average particle size, which is in the range of 0.5 to 100 µm.

Preferred embodiments are products as specified herein wherein the wax dispersion has a high viscosity, e.g. a viscosity of 1,000 mPas or higher.

Further in particular, the present invention is concerned with products that comprise an applicator whereto a wax dispersion has been applied, wherein the particles in the wax dispersion have a particle size which is in the range of 0.5 to 100 µm and wherein the wax dispersion comprises
(a) a wax phase having a melting point which is equal or higher than 25 °C, that contains at least one oil or wax component selected from dialkyl(ene) ethers, dialkyl(ene) carbonates, dicarboxylic acids or hydroxyfatty alcohols or any mixture of these substances and at least contains one emulsifier, and
(b) an aqueous phase.

The wax dispersions contain
(a) 1- 75 w/w-% of a wax phase having a melting point higher than 25 °C, that contains at least one oil or wax component selected from dialkyl(ene) ethers, dialkyl(ene) carbonates, dicarbonic acids or hydroxyfatty alcohols or any mixture of these substances and contains at least one emulsifier, and
(b) 25 - 99 w/w-% of an aqueous phase, relative to the total weight of the complete composition.

Preferred wax dispersions contain 5 - 30 weight-% of the above mentioned wax phase; particularly preferred wax dispersions contain 10 - 25 weight-% of the wax phase relative to the total weight of the wax dispersion.

The products of the invention can be made by applying a wax dispersion to an applicator as a finely dispersed composition. They can be applied in a cold process which is more convenient than when using waxes which need to be applied as a melt and subsequently cooled. They as well can be applied as concentrates with low water content, for example with a water content of only 25 weight-%, or as highly diluted formulations, e.g. having a concentration of only 1 weight-%.

Particular embodiments of this invention are products with a wax dispersion having wax particles comprising a combination of wax components selected from dialkyl(ene) ethers, dialkyl(ene) carbonates, dicarbonic acids or hydroxy fatty alcohols and any mixtures thereof. These particular wax dispersions have excellent sensorial properties, good caring properties and are characterized by their less 'heavy' and less greasy and dry skin feel.

In still a further aspect this invention relates to products that comprise an applicator whereto a wax dispersion and an additional aqueous phase have been applied. The wax dispersion and additional aqueous phase may be applied to the applicator sequentially or simultaneously.

The present invention further concerns products that comprise an applicator whereto a wax dispersion has been applied and which products have been dried.

In a different aspect, this invention relates to products that comprise an applicator whereto a wax dispersion has been applied and which products have been dried, and which products therefore are dry or essentially dry. In a further aspect this invention relates to products that comprise an applicator whereto a wax dispersion and an additional aqueous phase have been applied and which products have been dried subsequently, and which products therefore are dry or essentially dry. 'Dry' refers to the situation where the water content is very low, i.e. lower than 1 % and 'essentially dry' means that the product contains limited amounts of water, e.g. less than 10 % of the total weight of the product, preferably less than 8 %, more preferably less than 5 %, still more preferably less than 2 %.

In one embodiment the invention concerns a product comprising an applicator to which an additional aqueous phase has been applied, after which the product is dried, and to which subsequently a wax dispersion is applied. If desired, the thus obtained product may in turn be dried.

A particular embodiment concerns a product comprising an applicator to which an additional aqueous phase has been applied and to which subsequently a wax dispersion is applied, whereafter the product is dried. Another embodiment concerns a product as defined herein comprising an applicator to which a wax dispersion has been applied, whereafter the product is dried, and to which subsequently an additional aqueous phase is applied, and if desired, whereafter the thus obtained product is dried.

In a further aspect there is provided a method of manufacturing a product as described herein according to the features of claims 16.

In a preferred method of manufacturing, said applicator is coated with a wax dispersion. In an alternative method of manufacturing, the applicator is sprayed or impregnated with a wax dispersion.

In another aspect there is provided a method of manufacturing a product as described herein, said method comprising applying to the applicator a wax dispersion and an additional aqueous phase, either subsequently or simultaneously. In a preferred method of manufacturing, said applicator is coated with a wax dispersion and sprayed or impregnated with an additional aqueous phase.

In still a further aspect there is provided the use of a product as described herein as a cleansing tool, in particular in personal care applications.

### Detailed Description of the Invention

The applicator in the products according to this invention can be resilient or non-resilient. The applicator can be used as such or can have a suitable handle. One or more of the outer sides of the applicator may be made of different materials having different properties. For example one side may be soft while another side is rougher. The latter side can be abrasive, it can be used for rubbing or scouring.

The applicators can be hard, soft, semi-soft, resilient or not, squeezable or not.

One type of embodiments are puffs (poufs), pads, brushes, gloves, mitts, swabs or cotton balls.

Another type of embodiments are sponges. Sponges comprise sponges as such, foams and felts, composed of synthetic and/or natural materials

Still another type of embodiments are bars.

For convenience of use, the applicators may have a suitable handle. Embodiments of such applicators have a pad, puff or sponge portion that preferably is resilient and a finger grip portion. One type of such applicators are those having a generally T-shaped configuration. Examples of such applicators comprise resilient discs with a small upstanding handle element.

The applicators can be made of materials which are capable of holding, adsorbing or absorbing a lipid and an aqueous phase. Preferably, the applicator material is structured such that it is porous or absorbent in nature. The latter can be due to the chemical structure of the applicator materials or their physical arrangement or both. Examples of particular physical arrangements are porous structures, or cellular or microcellular structures.

The applicators can be made of one type of material or from different materials that can be arranged in different manners along the applicator. Small portions of one or more materials of different or equal size may be incorporated into a matrix of the same or another material. Or the applicators can be multilayered such as a stack of layers or concentric layers or they can be of one type of material. Applicator parts, either or not made of different materials can be linked together by gluing, sewing, stitching or any other technique known in the art.

In one group of embodiments the applicator comprises a core which is partially or completely wrapped in a layered material. The wrapping material may be the.same or different from the material or materials used in the core.

The materials of which the applicators are made in particular are polymeric and may be both from natural and non-natural origin. There can be one or more polymeric materials that may be cross-linked or not. Optionally other non-polymeric materials such as binders, fillers, dyes and the like, may additionally be present.

The materials can be more or less inert or they can be decomposable, in particular they can be biodegradable. The materials may also be flushable. As used herein, by 'flushable' is meant that the material will pass through at least 3 meters of.waste pipe in two toilet flushes.

Examples of polymeric materials of which the applicators are composed are non-natural polymers such as polyethylene, polypropylene, PET, polyamide, polyvinyl alcohol, polyurethane, and the like, and natural or natural-derived polymers such as cellulose, wood pulp and the like, and mixtures of such synthetic and natural fibres or materials.

Where the applicator is in the form of a puff (pouf) it can be composed of spongy or resin foamy materials, optionally wrapped in a suitable mono- or multilayered material, which can be made of a closed or an apertured material layer or film. In other embodiments the puff is made of one or more layers of material that can be bound or glued together in the core of the puff.

Where the applicator is in the form of a bar it may be composed of waxy material in solid state, optionally in admixture with other ingredients. Preferably, such embodiments are wrapped in a suitable layered wrapping material which may hold the aqueous phase or the wax dispersion while the other phase is kept inside the bar as depot in the core.

The bar may be apertured, having small cavities which may hold particular ingredients, also including the aqueous phase which thus is entrapped in the bar.

Applicators in the form of bars may be designed such that the bar slowly decomposes or dissolves during use, e.g. by body heat or by any other external factor. In particular, the bar may be composed of a solid material which decomposes or dissolves during use, e.g. due to body heat.

If layered materials are used, these materials in themselves may be mono or multilayered, woven or non-woven. They can be made of one or of several materials. Particularly preferred layered materials are non-woven materials that have a web structure of fibrous or filamentous nature, in which the fibres or filaments are distributed randomly or with a certain degree of orientation, the former being obtainable by air-laying or certain wet-laying processes, the latter in other wet-laying or in carding processes. The fibres or filaments can be natural, for example wood pulp, wool cotton, linen and the like, or synthetic, for example polyvinyls, polyesters, polyolefins, polyamides and the like.

Two different types of non-woven materials can be distinguished, based on the raw material that has been used.

A first type of layered materials is paper based. The raw materials for these layered materials are made almost exclusively of cellulose-based fibres or filaments from plant cellular sources (pulp). Where high wet strength or firmness of the layered material is desired, binding materials can be added, e.g. the so-called 'wet strength resins'. Softness can be increased by adding additives. In a second type use the web is made mainly of staple fibre, e.g. based on cotton, wool, linen and the like.

Usually, non-woven materials for use in the applicators of the invention are made of cellulose fibres, synthetic fibres or mixtures of both. Polyester, polyethylene and polypropylene are known as suitable polymers for the preparation of synthetic fibres. Also in these products binders can be used to increase the firmness of the non-woven fabric.

Webs of increased strength can be obtained by using the so-called spunlace or hydro-entanglement technique. In this technique the individual fibres are twisted together so that an acceptable strength or firmness is obtained without using binding materials. The advantage of the latter technique is the excellent softness of the non-woven material.

Other non-woven materials are made of a mixture of pulp and staple fibre and are available with binding materials, in particular those mentioned above, or without binding materials. In the latter instance the non-woven is preferably made by the spunlace or hydro-entanglement procedure.

In a preferred embodiment of the present invention, the layered material is made of cellulose pulp with a small amount of binding material. The amount of binder in the carrier material is in the range of 5 to 20 % (w/w).

In a particularly preferred embodiment the non-woven fabric is prepared by the water entanglement procedure and does not contain binding material.

The selection of the raw material of which the non-woven layer material is made depends on the manufacturing procedure. Typically in the manufacture of non-woven layered materials by the hydro-entanglement process, use is made of mixtures of cellulose fibres and synthetic fibres. The relative quantity of synthetic fibres in the non-woven fabric is from 0 to 100 % and preferably is between 10 and 70 %, more preferably in the range of 30 to 50 % (all percentages being w/w).

### Applicator and Wax Dispersion

According to this invention the applicator is contacted with a wax dispersion. In some embodiments the applicator is contacted with another phase, which may be a polymeric phase.

The wax dispersion may be applied to the whole applicator, i.e. continuously, or to parts of the applicator, i.e. discontinuously. Where more than one wax dispersion is applied, one of these may be applied continuously while another is applied discontinuously. The wax dispersion or dispersions, can be applied at the surface or in the internal of the applicator. If applied at the surface, the wax dispersion can be present at one side or at several sides of the applicator; or one wax dispersion may be present at one side while the other wax dispersions may be present at one or more of the sides of the applicator.

In the instance where one or more wax dispersions are applied discontinuously, they may be present in or at certain areas, in particular in or at one or more areas of the applicator. In that instance, the wax dispersion(s) may be present as one or more forms or shapes. For example they can be present as dots or spots, lines or stripes, as geometrical figures such as squares, rectangles, circles and the like, as symbols such as letters, text, logos, figures and the like, or as trademark signs, or any other such forms, or a combination thereof. The forms or shapes may be present over the entirety of the applicator or grouped in one or more areas, for example in a corner or in the centre area.

In one type of embodiments, one wax dispersion is applied on one or on several sides of the applicator in the form of stripes, dots or other forms covering the entire surface or only a part of the surface of the applicator. In further embodiments, one or more other wax dispersions can be added. ,

This may be done in a second step preferably after the application of the first wax dispersion or simultaneously in a one step operation.

Different parts of the applicator may contain different wax dispersions. For example, the applicator may at one side contain one wax dispersion and at another side another wax dispersion.

Or the applicator may be composed of two or more parts that are linked together, each part having been treated with a different wax dispersion. This may result for example in an applicator having at one portion different properties than at another portion.

Where the applicator is in the form of a puff, a pad, mitt, glove, or a sponge it may be coated with a wax dispersion or the puff may have a wax dispersion deposed at the inner portion of the applicator. If deposed at the inner portion, the wax dispersion may be distributed homogeneously, meaning that is distributed over the whole inside in more or less equal quantities, or inhomogeneously.

Where the applicator is in the form of a bar or sponge, it can be wrapped into a sheet of material to which a wax dispersion may be applied. Furthermore, the bar or sponge material itself may contain the same or different wax dispersion(s). The wax dispersion at the inner portion of the applicator may have been deposited or the applicator may have been impregnated with a wax dispersion. Or the bar or sponge may contain pellets, beads, capsules, or the like containing the wax dispersion, which are dissolved during use. Such pellets, beads or the like are made of a suitable material that dissolves during use, e.g. a water-soluble or -degradable polymer. Another embodiment are bags made of (non-)woven or apertured film material, which contain beads, pellets, capsules, or the like that contain the wax dispersion.

Where the applicator is in the form of a bar, it may be apertured, having a plurality of cavities that may contain a wax dispersion.

Where the applicator is in the form of a sponge, it may be made of a decomposable material such as a biodegradable material. For example, it can be made of dissolvable cellulose, which can be mixed with a wax dispersion when the cellulose is still in a liquid state during the production process.

In other embodiments, there is provided an applicator whereto a wax dispersion and an additional aqueous phase have been applied sequentially or simultaneously. Said additional aqueous phase may be water or a water-based formulation containing other ingredients, in particular the ingredients mentioned hereinafter.

In certain types of.such embodiments, the applicator is first contacted with an additional aqueous phase and subsequently with a wax dispersion.

In other types of embodiments, the applicator is contacted with a wax dispersion and subsequently with an additional aqueous phase.

The additional aqueous phase can also be applied at various stages in the production process of the end product, including during the production of the applicator material.

In the embodiments containing both an additional aqueous phase and a wax dispersion, the wax dispersion as well as the additional aqueous phase may be applied as described above; i.e. either the wax dispersion or the additional aqueous phase, or both, may be applied in an equal manner or differently, and may be applied continuously or discontinuously, at the surface or the interior of the applicator, at one or at several sides, in certain areas and/or as certain shapes.

Different parts of the applicator may contain different wax dispersions and aqueous phases. For example the applicator may at one side contain a wax dispersion and at another side an aqueous phase.

Where the applicator is composed of two or more parts that are linked together, each part may have been treated with a different phase, i.e. one part with a wax dispersion and another with an aqueous phase. This may result for example in an applicator that at one portion has cleansing capacity and at another portion has caring capacity.

Where the applicator is in the form of a bar or sponge, it can be wrapped into a sheet of material to which a wax dispersion may be applied. As mentioned before, the bar or sponge material itself may contain the same or a different wax dispersion. This type of applicators may further contain an aqueous phase which may be at the surface layer or at the inside.

In still a further aspect, this invention provides dry or essentially dry products, or products with limited water content. Such products can be obtained by applying a wax dispersion of low water content or by application of an appropriate drying step. A wax dispersion of low water content may contain less than 20 % (w/w relative to the total weight of the wax dispersion) or less than 10 %. Multiple variants are possible :
a) The applicator is treated with a wax dispersion of the invention and subsequently dried.
b) The applicator is treated with an additional aqueous phase, treated with a wax dispersion and subsequently dried.
c) The applicator is treated with an additional aqueous phase, dried, treated with a wax dispersion, and optionally dried; the latter drying step can be discarded if a wax dispersion of low water content is applied.
d) The applicator is treated with a wax dispersion, an additional aqueous phase and dried.
e) The applicator is treated with a wax dispersion, dried, subsequently treated with an additional aqueous phase and the whole is optionally dried.

Preferably the product is dried after the additional aqueous phase has been applied. Since the additional aqueous phase can be applied at various pints in the production of the end product, the drying step can also occur at various points in the production process. The product can also be dried after application of a wax dispersion.

In each drying step, drying may be performed to a different extent, i.e. partial drying or complete drying.

Further possible variants are those wherein multiple additional aqueous phases and/or dispersions are applied, followed by an appropriate drying step or multiple drying steps.

Also included is the possibility to apply multiple additional aqueous phases and multiple wax dispersions and to introduce several drying steps. In each step, it is possible that the phase is applied to only a portion of the applicator, or to one side of the applicator, or to several sides. Any combination of such applications of the phases are deemed within the ambit of the present invention.

Thus in a particular group of embodiments the invention relates to products that are dry or essentially dry. 'Dry' refers to the situation where the water content is very low, i.e. lower than 1 %. As used herein 'essentially dry' means that the product contains limited amounts of water, e.g. less than 10 %,of the total weight of the product, preferably less than 8 %, more preferably less than 5 %, still more preferably less than 2 %. More generally 'essentially dry' means that after manufacture, no water or aqueous-based lotion is added to the applicator. As used herein, a % is w/w to the total weight of the applicator with all materials incorporated therein or thereon.

### The wax dispersion

The products according to the invention contain a wax dispersion, which is composed of an aqueous dispersion of wax particles. The latter in turn are composed of a wax material, herein referred to as the 'wax phase' and are dispersed in an aqueous medium, herein referred to as the 'aqueous phase'. Hence the products of the invention carry a two phase system comprising a lipid and an aqueous phase.

As used herein, the term 'wax' refers to any natural or synthetic components or component mixtures having the following properties: a consistency which ranges from solid to semi-solid (creamy), from coarsely crystalline to microcrystalline or even amorphous, from transparent to opaque, having a relatively low melting point, being of relative low viscosity at temperatures slightly above their liquefying point, being oil soluble and having a melting point or range of above 25 °C. Waxes can be composed of one or more components, synthetic as well as natural, and can in principle be composed of any oil soluble material having a waxy consistency, including mixtures thereof. Waxes may also contain amounts of components with a lower melting point or liquid components as long as the melting point or range of the total composition of the wax is above 25 °C.

The wax phase has a melting point or a melting range above 25 °C, in particular in the range of 30 to 75 °C, more in particular in the range of 35 to 50 °C, or of 30 to 45 °C, or of 35 to 45 °C, preferably in the range of 32 to 40 °C or of 37 to 42 °C. More preferably the melting temperature or melting range is above human body temperature. Most preferably the melting temperature or melting range approximates or is equal to human body temperature.

In many instances, upon usage' of the products of this invention, the wax phase and the aqueous phase in the wax dispersion undergo some form of interaction, which may contribute to the beneficial properties of the products. The selection of the melting point or range of the wax phase may be critical for such interaction, but also the addition of certain components to either the wax phase or the aqueous phase or to both may contribute thereto. A number of such additional components are described hereinafter in more detail.

In some embodiments of this invention the wax phase in the wax dispersion may have a relatively higher melting point or range. The melting point or range may for example be higher than body temperature, e.g. higher than 40 °C, or higher than 45 °C. Upon application of such products, a more intense interaction between the two phases of the wax dispersion may be required, or the application of higher temperatures, to promote the interaction. In the latter instance the consumer may, for example, be required to contact the product first with hot water and then to apply it. In the former instance the aqueous phase may contain agents that promote a stronger interaction of the aqueous phase with the wax phase.

As used herein the term 'melting range' refers to a temperature range that starts from the temperature at which a substance or composition loses its solid,consistency up to the temperature where it becomes completely liquid. A melting range is considered to, be within a defined temperature range when it overlaps with that defined temperature range, or should be considered to be above a specified temperature when the range is above said temperature.

As used herein 'ambient temperature' or 'room temperature' refers to a temperature that is in the range of about 20 to about 25 °C.

The wax phase can further contain small amounts of water although the water content of the wax phase generally is low. In preferred embodiments of the invention, the wax phase contains less than 10 weight % of water, the water content in particular is lower than 6 weight %, more in particular the water content is lower than 3 weight-%. In preferred embodiments, the wax phase is water free. As used herein, 'water free' means that the phase is composed of materials of low water content to which no water has been added.

The wax phase in the wax dispersion can change to another state in that during storage, or upon usage by the consumer the wax phase becomes semi-solid or liquid. This change of state may be induced by physical factors, such as temperature or pressure, .. but may also be induced by chemical factors, such as particular chemical components or by a photochemical reaction.

In particular embodiments, multiple wax dispersions; i.e. wax dispersions made of phases of different composition, may be applied to the applicator. For example, one type of wax dispersion is applied to one side of the applicator while another type is applied to another. Each of these wax dispersions may or may not contain one or more of the ingredients mentioned hereinafter, for example one or more ingredients selected from the active ingredients, the dyes, emulsifiers, and other ingredients mentioned hereinafter. In case of various dyes, multi-colored patterns may exist, for example, each wax dispersion may have a different color or may be uncolored.

In other particular embodiments, the wax dispersion may be composed of two or more different wax phases, i.e. contain wax particles composed of different wax phases. The size, composition and properties of the wax particles may differ and may be selected in function of the envisaged properties and uses of the end product.

The wax dispersions further contain an aqueous phase. The aqueous phase contains water and, optionally, other ingredients, e.g. ingredients that have a stabilizing effect on the wax dispersion. The aqueous phase can contain water-soluble actives, water soluble, dispersible or gellifying polymers, humectants etc. Furthermore, the aqueous phase in the wax dispersions may contain any of the ingredients mentioned hereinafter as possible ingredients of the 'additional aqueous phases',

The wax dispersions may be concentrates, i.e. having low content of water or may be diluted with water. Where the wax dispersions are concentrates their viscosity usually will be higher and such dispersions will have a more creamy or pasty consistency. Wax dispersions with higher viscosity may also be obtained by adding specific amounts of suitable thickeners. Wax dispersions of higher viscosity may be preferred in certain techniques for applying the wax dispersions to the applicator.

Preferred embodiments are products having a wax dispersion of high viscosity, e.g. the wax dispersion has a viscosity of 1000 mPas or more, or 5000 mPas or more, or 10,000 mPas or more, or 20,000 mPas or more. Although it is possible to use dispersions of very high viscosity, for practical reasons the viscosity of the wax dispersions by preference will not exceed 100,000 mPas. A particular useful range is between 10,000 and 100,000 mPas, preferably between 20,000 and 50,000 mPas.

The wax particles in the wax dispersions preferably are of sufficiently small size to ensure a stable dispersion. In general the average size of these particles is in the range of about 0.5 to about 100 µm. Of particular interest for the invention are wax dispersions wherein the average particle sizes are in the range from 1 - 50 µm and in particular from 5 - 30 µm.

The wax dispersion may be applied differently at the different sides of the applicator. For example one side may completely be covered, while on another side the wax dispersion may be applied in a pattern, e.g. as stripes.

### The Wax Phase

The wax phase comprises one or more components that are designated as 'waxes', which are materials of waxy consistency as outlined above. Waxes are materials that have a solid to semi-solid (or creamy) consistency, crystalline or not, being of relative low viscosity a little above their liquefying point. Waxes can be composed of one or more components, synthetic as well as natural, and can in principle be composed of or comprise any oil soluble material having a waxy consistency, including mixtures thereof.

Waxes which can be used may be synthetic or natural waxes, as well as other oil soluble materials that have a waxy consistency. Waxes are dialkyl(ene) ethers, dialkyl(ene) carbonates, dicarboxylic acids and derivatives (in particular the C₁₆-C₄₀-dialkylesters of dicarboxylic acids, e.g. the C₁₆-C₄₀-alkyl stearates, C₁₈-C₃₈-alkylhydroxystearyl stearates or C₂₀-C₄₀-alkyl erucates) and hydroxy fatty alcohols.

Any of these wax phases may contain homologous components that are liquid, as long as the total composition making up the wax phase has a waxy consistency. For example, waxy fats may contain oils, waxy fatty alcohols may contain liquid fatty alcohols, etc., in such amount that the total composition has a waxy consistency and in particular has the melting point or range specified above.

### Wax Components

Natural waxes comprise waxes from vegetal origin, such as purcelline, shea butter, cocoa butter, Japan wax, esparto gras wax, cork wax, guaruma wax, rice shoot wax, ouricury wax, montan wax, sunflower wax, ceresine wax, sugar cane wax, carnauba wax, candelilla wax, lanolin, fruit-derived waxes, such as orange wax, lemon wax, grapefruit wax and bayberry wax, and the like, and of animal origin such as beeswax, woolwax, spermateci and bear fat, shellac wax, and the like. Natural waxes further comprise mineral waxes such as ceresine and ozokerite waxes. Preferred among these natural waxes are waxes of vegetal origin. Synthetic waxes comprise petroleum-based waxes such as paraffin, vaseline, petrolatum, micro wax. Further synthetic waxes are polyalkylene and polyethyleneglycol waxes, e.g. polyethylene wax; waxes based on chlorinated naphtalenes such as 'Halowax', synthetic hydrocarbon waxes, and the like, including mixtures thereof. Further waxes are chemically modified waxes, in particular hardened or hydrogenated waxes such as, for example, Montan-ester waxes, Sasol waxes and hydrogenated jojoba waxes.

Other wax components may be selected from the esters from aromatic carbonic acids, dicarboxylic acids, tricarboxylic acids and hydroxycarboxylic acids (e.g. 12-hydroxystearic acid ) and saturated and/or unsaturated straight or branched alcohols, and further lactides of long-chain hydroxy carbonic acids. Examples are C₂₀-C₄₀-dialkylesters of dimeric acids, C₁₈-C₃₈-alkylhydroxystearoylstearates or C₂₀-C₄₀-Alkylerucates. Myristyl lactate is particularly attractive for use on wipes for skin care, because of its good affinity to the skin. As further useful components there can be mentioned silicone waxes.

Further wax components that can be used are C₃₀-C₅₀-alkyl bees wax; tri-C₁₆-C₄₀-alkyl citrates; e.g. tristearyl citrate, triisostearyl citrate, trilauryl citrate; ethyleneglycol difatty acid esters, in particular the ethylene glycol di-C₁₂-C₃₀-fatty acid esters, e.g. ethylene glycol dipalmitate, ethyleneglycol distearate, ethyleneglycol di(12-hydroxystearate).

In preferred embodiments, the wax phase comprises any of the components designated herein as 'waxes' and further components, which may be selected from C₁₂-C₂₄-fatty alcohols, mono-, di- or tri-esters of glycerine and C₁₂-C₂₄-fatty acids, mono- or diesters from ethylene glycol and C₁₂-C₂₄-fatty acids or any mixtures of these components.

The total amount of waxes in the wax phase in particular is at least 50% (e.g. from 50-99.9%, or from 50-99%), preferably at least 70% (e.g. from 70-99.9%, or from 70-99%), more preferably at least 90% (e.g. from 90-99.9%, or from 90-99%), w/w of the total amount of components making up the wax phase.

In a particular embodiment, the total amount of waxes in the wax dispersion is in the range of from 0.1 to 45%, preferably of from 5 to 30%, more preferably of from 10 to 25%, all % being w/w relative to the total weight of the wax dispersion.

In a particular aspect of this invention there are provided products as specified herein wherein the wax phase essentially consists of one or more waxes selected from the waxes mentioned herein, including mixtures thereof. The waxes can be present in various amounts, e.g. the amounts mentioned hereinabove or hereinafter.

### Fats and oils

The wax phase may comprise fats and also certain amounts of oils: When present, the amount of oils will always be such that the wax phase is sufficiently solid as to form a dispersion and in particular will have the melting points or melting ranges outlined above. Fats or oils which can be used in the wax phase comprise natural fats or oils, or natural fat or oil derivatives, in particular of vegetable origin. Examples are almond oil, soybean oil, sunflower oil, safflower oil, corn oil, kernel oil, canola oil, borage oil, evening primrose oil, grapeseed oil, wheat germ oil, avocado oil, jojoba oil, sesame oil, walnut oil, linseed oil, palm oil, olive oil, macadamia oil, castor oil, rapeseed oil, peanut oil, coconut oil, and turnip seed oil, and in particular the hardened derivatives thereof. The latter are obtained by hydrogenation of fats or oils. Preferred are hardened oils or fats from vegetal origin, e.g. hardened peanut oil, soybean oil, turnip seed oil, cotton seed oil, sunflower oil, kernel oil, linseed oil, almond oil, corn oil, olive oil, sesame oil, cocoa butter, shea butter and coconut oil, in particular hardened castor oil or palm oil.

The wax phase may further comprise fatty components isolated from these natural oils, i.e. pure triglycerides or mixtures thereof, or the latter components having been prepared by chemical synthesis. These so-called trigycerides (or triacyl glycerines) are esters of glycerines with fatty acids or fatty acid mixtures The latter also include technical mixtures obtained by hydrolysis from oils or fats, or from fractions thereof, or by.fractioning fatty acid mixtures after hydrolysis.

The fatty acids in said triglycerides may be saturated or unsaturated, straight or branch chained, substituted or unsubstituted. Preferred triglycerides are those glycerine esters derived from fatty acids, either saturated or unsaturated, having from 10 to 60, in particular from 12 to 36, more particularly from 12 to 24, preferably from 16 to 20 carbon atoms. Preferred such fatty acids are, for example, palmitic, palmic, oleic, lauric, myristic, stearic, hydroxystearic, behenic acid, or mixtures thereof. Amongst the triglycerides those derived from saturated fatty acids are of particular interest.

Preferred among the triglycerides are those that liquefy at temperatures which are in the range of 35-50°C , preferably from 35-45°C, more preferably from 37-42 °C. Further preferred are triglycerides that contain saturated, unbranched and unsubstituted fatty acid residues. They can be mixed esters, i.e. tri-esters of glycerine with different fatty acids. Suitable triglycerides that can be incorporated are hardened castor oil, a triester from glycerine and a hydroxystearic acid, a product that is commercially available under the tradename Cutina HR^{™}. Further such triglycerides are glycerine tristearate, also referred to as stearin, glycerine tribehenate, glycerine tripalmitate, glycerine trilaurate, glycerine trioleate, glycerine trimyristate.

The wax phase may also contain mono- or diglycerides, optionally in mixture with the fats and oils mentioned herein, in particular with triglycerides. The mono- or diglycerides for use in the wax phase are derived from saturated or unsaturated, linear or branch chained, substituted or unsubstituted fatty acids or fatty acid mixtures. Also, in this instance the melting point or melting range of the wax phase preferably is as mentioned above, in particular is above ambient temperature, further in particular above 25 °C, and more in particular is in the range of 32 °C to 40 °C. Particular mono-or diglycerides are mono- or di-C₁₂- C₂₄ fatty acid glycerides, specifically mono- or diC₁₆- C ₂₀ fatty acid glycerides, for example glyceryl monostearate, glyceryl distearate, Mixtures of mono-, di- and, optionally, triglycerides can be derived from fractions of fatty acids. An example of such mixture for use as a component of the wax phase is a mixture of C₁₂- C ₁₈ mono-, di- and triglycerides.

In particular embodiments of the invention, the wax phase contains mixtures of mono-. di- and triglycerides. Examples of such glyceride mixtures are mixtures of C₁₂-C₁₈-mono-, di- and triglycerides or stearic acid mono-, di- and triglycerides. Mixed esters as well as mixtures of mono-, di- and triglycerides are of particular interest because of their low propensity to crystallize and their capacity to improve the consistency of the formulation making up the wax phase.

The wax phase may also comprise alkyl esters of fatty acids, wherein the alkyl group in the latter has from 1 to 30 carbon atoms, preferably from 12 to 24 carbon atoms. The fatty acids in said alkyl esters in particular are C₁₂-C₃₀ fatty acids, more in particular C₁₂-C₂₀ fatty acids. The alkyl groups in said esters preferably are derived from fatty alcohols as well as of mixtures thereof, which, for example, are obtained by high pressure hydrogenation of technical mixtures of the methyl esters derived from fats or oils.

Preferred are the alkyl esters of C₁₆- C ₂₄ fatty acids, more preferably from C₁₆- C ₁₈ fatty acids, and C₁- C ₃₀ fatty alcohols, preferably C₈- C ₂₄ fatty alcohols, more preferably C₁₂- C ₂₀ fatty alcohols. Examples are the C₁₆-C₄₀-alkyl stearates, in particular the C₂₀-C₄₀-alkyl stearates.

Of particular interest in this regard are, e.g. stearyl stearate, palmityl stearate, stearyl behenate, cetyl stearate, cetyl behenate, cetyl palmitate, cetearyl behenate, behenyl behenate, stearyl heptanoate, stearyl octanoate, myristyl myristate, myristyl palmitate, myristyl stearate, myristyl isostearate, myristyl oleate, cetyl isostearate, cetyl oleate, stearyl isostearate, stearyl oleate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl isostearate, behenyl oleate, erucyl isostearate.

Of further interest are esters of linear C₆-C₂₂-fatty acids with branched alcohols, in particular 2-ethylhexanol, esters of branched C₆-C₂₂-fatty acids with linear alcohols, esters of C₁₈-C₃₈-alkylhydroxycarbonic acids with linear or branched C₆-C₂₂-fatty alcohols, esters of linear and/or branched fatty acids with poly-alcohols (e.g. propylene glycol, dimerdiol or trimertriol) and/or Guerbet alcohols, as well as esters of C₆-C₂₂-fatty alcohols and/or Guerbet alcohols with aromatic carbonic acids, in particular benzoic acid, esters of C₂-C₁₂-dicarbonic acids with linear or branched C₁-C₂₂ -alcohols (e.g. dioctyl malate) or C₂-C₁₀ -polyoles having 2 to 6 hydroxyl groups.

The wax phase may further contain other oily components, which are lipid components that are liquid at ambient temperature, in particular are liquid at 20 °C or at 25 °C. These components in particular are any of the oily components described hereinafter in more detail.

The oily components can be any of the oils mentioned hereinabove as 'oils and fats', in particular any of these that are liquid at ambient temperature, in particular are liquid at 20 °C or 25 °C. Of particular interest are the mono-, di- and triglycerides mentioned hereinabove that are liquid at ambient temperature, in particular are liquid at 25 °C. The oily components can further be fatty acids and fatty alcohols, described hereinafter in the respectively sections, that are liquid at 25 °C.

The wax phase can contain various amounts of fats and oils. In certain embodiments, the fats and in particular the glycerides are present in an amount of up to 60% (e.g. from 0.1 to 60%), or in other embodiments up to 70% (e.g. from 0.1 to 70%), or up to 80% (e.g. from 0.1 to 80%) (w/w), relative to the total amount of the wax phase. In other embodiments, e.g. in those wherein the wax phase contains other components of waxy consistency, the glyceride mixture can be present in amounts in the range of 0.1-45 w/w %, preferably in amounts of 0.1-15 w/w % and most preferable 1-12 w/w % relative to the total weight of the wax dispersion.

On the wax phase containing dialkyl(ene)ethers or -carbonates, dicarboxylic acids or hydroxy fatty alcohols, the amount of said fatty ester glycerides can be up to 50% (e.g. from 0.1 to 50%) and more preferably up to 40% (e.g. from 0.1 to 40%) (w/w), relative to the total quantity of the wax phase, or in the lower ranges mentioned in the preceding paragraph.

The total amount of fats or oils, or of mixtures of fats and oils and/or oily components in the wax phase in particular is at least 90% (e.g. from 0.1 to 90%), more in particular at least 70% (e.g. from 0.1 to 70%), or at least 50% (e.g. from 0.1 to 50%), w/w of the total amount of components making up the wax phase.

### Fatty alcohols

The wax phase may also comprise fatty alcohols. Fatty alcohols that can be used are, for example, C₁₂-C₅₀-fatty alcohols, in particular the C₁₂-C₂₄-fatty alcohols, that are derived from natural fats, oils or waxes such as, for example, myristyl alcohol, 1-pentadecanol, cetylalcohol, 1-heptadecanol, stearylalcohol, 1-nonadecanol, arachidylalcohol, 1-heneicosanol, behenylalcohol, brassidylalcohol, lignocerylalcohol, cerylalcohol or myricylalcohol as well as Guerbet alcohols. Preferred for use in the present invention, are saturated, straight or branch chained fatty alcohols. However also unsaturated, straight or branch chained alcohols can be used, optionally in admixture with saturated alcohols. Preferably the alcohols will be selected such that the melting point of the mixture is as referred to hereinabove and more in particular is in the range of 32 to 40 °C.

Mixtures of fatty alcohols can evidently also be used, including fatty alcohol fractions obtained from the reduction of the corresponding fatty acid fractions derived from naturally occurring oils or fats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil.

Synthetic alcohols can also be used such as, for example, the linear fatty alcohols of an even number of carbon atoms resulting from the Ziegler-synthesis (Alfole^{®}) or the partially branched alcohols resulting from the Oxo synthesis (Dobanole^{®}).

A preferred embodiment according to the present invention is that wherein the wax phase contains at least one fatty alcohol, more preferably at least one C₁₄-C₁₈-fatty alcohol. Also preferred is a wax phase with at least one C₁₆-C₁₈-Guerbet alcohol.

The total amount of fatty alcohols in the wax phase may vary and depends on the desired properties of the wax phase. In a number of instances it is desirable to have a relative higher quantity of fatty alcohols in the composition, in particular said alcohols will be present in an amount of 50% (e.g. from 50 to 99.9%, or from 50 to 90%); preferably at least 70% (e.g. from 70 to 99.9%, or from 70 to 90%), more preferably at least 90% (e.g. from 90 to 99.9%, or from 90 to 99%), (all w/w) of the total amount of components making up the wax phase. In other instances, relatively lower amounts are desired, the total amount of the fatty alcohols present in the wax phase is in the range of 0.1 - 50%, in particular of 1 - 40%, preferably of 1 - 30% (w/w), more preferably of 1 - 20% (w/w), still more preferably from 1 -10% (w/w).

In one embodiment the total amount of fatty alcohols in the wax dispersion is in the range of from 0.1 to 45%, preferably from 1 to 25%, more preferably from 5 to 20%, w/w relative to the total weight of the wax dispersion.

In a particular aspect of this invention there are provided products as specified herein wherein the wax phase essentially consists of one or more fatty' alcohols, in particular those specified in this patent specification, including mixtures thereof. The fatty alcohols can be present in various amounts, e.g. the amounts mentioned hereinabove or hereinafter.

### Fatty acids

The wax phase may also contain C₁₄-C₄₀-fatty acids, including mixtures thereof. Of particular interest are the C₁₆-C₃₀-fatty acids. These comprise, for example, myristic-, pentadecanoic-, palmitic-, margaric-, stearic-, nonadecanoic-, arachic-, behenic-, lignoceric-, cerotic-, melissic-, erucaic-, elaeostearic-, oleic-, lonolenic-, lauric acid as well as substituted fatty acids, e.g. hydroxy-substituted fatty acids such as, for example, 12-hydroxystearic acid, and the amides or monoethanolamides of these fatty acids.

The total amount of the C₁₄-C₄₀-fatty acids present in the wax phase, relative to the total weight amount of the wax phase, is in the range of 0.1 - 30% or the range of 1 - 30 % (w/w), preferably of 1 - 20 % (w/w), more preferably from 1 -10 % (w/w).

In one embodiment the total amount of fatty acids in the wax dispersion is in the range of from 0.1 to 45%, preferably from 1 to 25%, more preferably from 5 to 20%, w/w relative to the total weight of the wax dispersion.

In a particular aspect of this invention there are provided products as specified herein wherein the wax phase essentially consists of one or more fatty acids, in particular those specified in this patent specification, including mixtures thereof. The fatty acids can be present in varying amounts, e.g. the amounts mentioned hereinabove or hereinafter.

### Dialkyl(ene) ethers or -carbonates, dicarboxylic acids or hydroxy fatty alcohols

The wax phase contains dialkyl(ene) ethers, dialkyl(ene) carbonates, dicarboxylic acids or hydroxy fatty alcohols, or mixtures thereof, which ethers, carbonates, acids or alcohols in particular are those described hereinafter.

In a particular aspect of this invention there are provided products as specified herein wherein the wax phase essentially consists of one or more dialkyl(ene) ethers or - carbonates, dicarboxylic acids or hydroxy fatty alcohols, including mixtures thereof.

The dialkyl(ene) ethers or -carbonates, dicarboxylic acids or hydroxy fatty alcohols can be present in various amounts, e.g. the amounts mentioned hereinabove or hereinafter.

The addition of dialkyl(ene) ethers or -carbonates, dicarboxylic acids or hydroxy fatty alcohols, including mixtures thereof to the composition of the wax phase allows to optimize the properties of the wax particles, in particular their sensorial properties, i.e. the products, as well as the skin after the products have been applied, have a less greasier feel and also a less dry skin-feel, while having excellent skin caring properties.

### Dialkyl(ene) ethers

The dialkyl(ene) ethers are symmetric or asymmetric, straight or branch chained, saturated or unsaturated. Preferred are waxy, saturated C₁₆-C₃₀-dialkylethers, in particular C₁₆-C₂₄-dialkylethers. More preferred are C₁₆-C₂₀-dialkylethers, and particularly preferred are distearylethers and dibehenylethers. Dialkylethers of shorter chain length can also be used such as, for example, di-n-octylether, di-(2-ethylhexyl)-ether, laurylmethylether or octylbutylether, didodecylether, under the condition that the complete composition of the wax phase has the desired melting point.

These ethers can be obtained from the appropriate fatty alcohols in the presence of an acid catalyst following art-known procedures. Typical examples are the products that are obtained by the etherification of capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleyl alcohol, behenyl alcohol, erucyl alcohol and brassidyl alcohol, Guerbet alcohols, as well as mixtures thereof, which, for example, are obtained by high pressure hydrogenation of technical mixtures of the methyl esters derived from fats or oils. Of particular interest are the dialkyl(ene) ethers that are solid at 25 °C.

### Dialkyl(ene) carbonates

The dialkyl(ene) carbonates are symmetric or asymmetric, straight or branch chained, saturated or unsaturated. Preferred dialkyl(ene) carbonates are waxy, linear or branch chained, saturated or unsaturated C₁₄-C₃₀-dialkyl(ene) carbonates. More preferred are C₁₆-C₂₄-dialkyl carbonates and amongst these the saturated linear C₁₆-C₂₂-dialkyl carbonates. Particularly preferred is distearyl carbonate. Also liquid dialkyl(ene) carbonates, such as, for example, dihexyl-, dioctyl-, di-(2-ethylhexyl)- or dioleylcarbonate, can be used, under the condition that the complete composition has the desired melting point.

These dialkyl(ene) carbonates can be obtained by re-esterification of dimethyl- or diethyl carbonates with the corresponding hydroxy compounds following art-known procedures. Typical examples of dialkyl(ene) carbonates are re-esterification products of dimethyl- and/or diethyl carbonate with capron alcohol, capryl alcohol, 2-ethylhexyl alcohol, caprinalcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleyl alcohol, behenyl alcohol, erucyl alcohol and brassidyl alcohol, Guerbet alcohols, as well as technical mixtures thereof, that.can be obtained by hydratation of methyl esters derived from suitable oils or fats or oil or fat fractions. Of particular interest are those dialkyl(ene) carbonates that are solid at 25 °C.

### Dicarboxylic acids

Dicarboxylic acids that can be used are, for example, C₉-C₃₄-dicarbonic acids. These comprise, for example, octadecanedioic acid, tetratridecanedioic acid, etc. Of particular interest are the azelainic acids, which are C₉-dicarboxylic acids.

### Hydroxy fatty alcohols

The hydroxy fatty alcohols for use in the wax phase compositions are saturated or unsaturated, straight chain or branched. Preferred are C₁₂-C₃₀-hydroxy fatty alcohols, at which the position of the hydroxy-substituent depends upon the synthesis route and the starting materials that have been used. Included are, for example, 1,10-decanediol, 1,2-hexadecanediol, 12-hydroxystearyl alcohol or hydroxy-Guerbet alcohols. Preferred are those hydroxy fatty alcohols that are solid at 25 °C, although liquid analogues can also be used, as long as the complete composition has the desired melting point. Particularly preferred is 12-hydroxystearyl alcohol.

The total amount of one or more of the dialkyl ethers, dialkyl carbonates, dicarbonic acids and the hydroxy alcohols present in the wax phase, relative to the total weight amount of the wax phase, is in the range of 0.1 - 30% or in the range of 1 - 30 % (w/w), preferably of 1 - 20 % (w/w), more preferably from 1 -10 % (w/w).

The total amount of dialkyl ethers, dialkyl carbonates, dicarbonic acids and the hydroxy alcohols is in the range of from 0.1 to 30%, preferably from 0.5 to 20%, more preferably from 0.5 to 10%, w/w relative to the total weight of the wax dispersion.

### Oil components

The wax phase may also comprise one or more oil components. The latter are components or component mixtures that are non water-mixable and that are liquid at ambient temperature, in particular are liquid at 20 °C or at 25 °C. These can be any liquid homologues of the components mentioned above as being waxes, e.g. liquid glycerides, hydrocarbons, silicone oils, ester oils and the like, but also liquid dialkyl(ene) ethers, dialkyl(ene) carbonates, dicarbonic acids and hydroxy fatty alcohols, and the like, as well as mixtures thereof. The total quantity of these oily components in the total composition of the wax phase preferably will be such that the wax phase is solid at room temperature, or that it has a melting point or range that is as specified hereinabove. In particular embodiments, the quantity of oil components in the wax phase is limited such that the melting point of the complete composition is above 25 °C.

The oil components will typically be present in quantities of less than 40% (e.g. from 0.1 to 40%) (w/w), in particular less than 30 weight % (e.g. from 0.1 to 30%), in particular less than 20 % (e.g. from 0.1 to 20%), or further in particular 0.1 - 15 %, more in particular from 0.5 - 10 % (w/w), preferably in quantities in the range of 2 - 10% or in quantities in the range of 0.5 - 5 w/w-%, relative to the total weight of the wax dispersion.

The oil components can be liquid mono-, di- or triglycerides; or mixtures thereof, in particular any of the above-mentioned oils of the glyceride type, that are liquid that are of natural or synthetic origin. Of particular interest are glycerides derived from C₆-C₂₄-fatty acids, in particular C₆-C₁₈-fatty acids, more in particular C₈-C₁₈-fatty acids. An example of such glycerides are the coco glycerides, a mixture of predominantly di- and triglycerides with C₈-C₁₈-fatty acids.

Further oil components that can be added are the liquid Guerbet alcohols based on fatty alcohols having 6 to 18, in particular 8 to 10 carbon atoms.

Further oily components, which can be used in the wax phase, comprise silicone oils, mineral and paraffin oils and synthetic oils, either aliphatic or aromatic, as well as mixtures thereof. Examples of such oils are squalane, squalene, isohexadecane, isoeicosane, polydecene, and also members of the group of dialkylcyclohexanes.

The wax phase may further contain silicone oils such as, for example cyclic silicones, dialkyl- or alkylarylsiloxanes, e.g., cyclomethicone, dimethyl polysiloxane and methylphenyl polysiloxane, as well as the alkoxylated and quaternized analogs thereof. Appropriate non-volatile silicone oils are e.g. polyalkylsiloxanes, polyalkylarylsiloxanes and polyethersiloxane-copolymers.

Still further oil components are from the ester type, in particular any of the liquid esters described above in the section 'oils and fats'. Of particular interest are liquid esters from linear, saturated or unsaturated C₆-C₂₂-fatty acids with linear or branched, saturated or unsaturated C₆-C₂₂-fatty alcohols respectively esters from branched C₆-C₁₃-carboxylic acids with linear or branched, saturated or unsaturated C₆-C₂₂-fatty alcohols.

Examples of oil components of the ester type are the following: decyl oleate, coco caprylate/-caprate (available under the tradename Cetiol^{®} SN), hexyl laurate, myristyl isostearate, myristyl oleate, cetyl isostearate, cetyl oleate, stearyl isostearate, isostearyl myristate, isostearyl palmitate, isostearyl stearate, isostearyl isostearate, isostearyl oleate, isostearyl oleate, oleyl myristate, oleyl isostearate, oleyl oleate, oleyl erucate , behenyl isostearate, erucyl isostearate, erucyl oleate. Further oil components are the esters from linear C₆-C₂₂-fatty acids with branched alcohols, in particular 2-ethylhexanol, esters from branched C₆-C₂₂-fatty acids with linear alcohols, esters from C₁₈-C₃₈-alkylhydroxycarboxylic acids with linear or branched C₆-C₂₂-fatty alcohols, esters from linear and/or branched fatty acids with multifunctional alcohols (e.g. propylene glycol, dimerdiol or trimertriol) and/or Guerbet alcohols, as well esters from C₆-C₂₂-fatty alcohols and/or Guerbet alcohols with aromatic carboxylic acids, in particular benzoeic acids, esters from C₂-C₁₂-dicarboxylic acids with linear or branched alcohols having 1 to 22 carbon atoms (e.g.. dioctyl malates).

Still further oil components are any hydrocarbons that are liquid at 20 °C of natural or synthetic origin, that are either aliphatic or aromatic, such as, for example squalane, squalene, paraffin oils, isohexadecane, isoeicosane or polydecene as well as dialkylcyclohexane.

### Further components

The wax phase may contain further components, which may be of waxy nature or otherwise. The use of these further components allows to influence the sensorial properties as well as the stability of the compositions, in particular after application to wipe material and more in particular to stabilize the wax particles being in contact with the aqueous phase, and to adapt the desired release profile. Any further components may also be added to influence consistency, feel and appearance. These components will generally be insoluble or poorly soluble in water. Water-soluble components can also be included, typically in combination with a solubilizing or emulsifying agent and some water.

Examples of further components are superfatting agents, thickeners, polymers, active ingredients, powders or powdered ingredients, film forming agents, UV-filters, anti-oxidants, hydrotropic agents, preservatives, insect repellents, self-tanning agents, solubilizers, perfume oils, dyes, etc.

The further components can be present in the dispersions in a total amount ranging from 0.1 - 45 weight-%, preferably from 5 - 30 weight-% and more preferably from 10 - 25 weight-%, relative to the total weight of the wax dispersion.

The wax phase may further contain consistency agents there such as, for example, small amounts of alkali metal or alkaline earth metal as well as aluminium salts of C₁₂-C₂₄-fatty acids or C₁₂-C₂₄-hydroxyfatty acids; preferred being calcium-, magnesium-, aluminium- and in particular zinc stearates.

The wax phase may further contain disintegrating agents, which are agents that cause a disintegration of the physical integrity of the wax phase.

Suitable disintegrating agents are agents that are subject to physical or chemical interactions either by auto-interaction or by interaction between two agents. This results in a physical or chemical interaction with the wax phase. One type of disintegrating agents are those that release a gas, e.g. by decomposition or by chemical reaction between two components. An example of a disintegrating agent is a solid mixture of a bicarbonate and an acid such as sodium or potassium carbonate with a suitable organic acid, e.g. citric acid. Upon contact with water, e.g. upon contact with the aqueous phase, the disintegrating components will interact and liberate carbon dioxide which physically alters the wax phase. Such physical alteration may, for example, cause the wax particles to become homogeneously distributed on the applicator. This may positively influence any interaction between the aqueous phase and wax particles, which in turn may have a positive effect on the transfer to the skin of materials, e.g. active ingredients, in these phases. Another reason for using a disintegrating agent can be the altered visual aspect of the wax dispersion.

The wax phase may further contain components that are subject to a polymerization reaction either during or after application on the applicator material. Examples of such components are oligomers that during or after application on the applicator continue to polymerize with monomers or other oligomers. Other examples are agents that cause netting or co-polymerization. There can also be agents that inhibit polymerization for a specific period of time. Alternatively there can be agents that accelerate polymerization e.g. under influence of external factors such as heat, light or pressure.

The wax dispersion may further contain dyes, either in the wax phase or in the aqueous phase or in both, that upon usage of the product change color due to a change of temperature or pressure. This will give the consumer a level of comfort and trust that the product delivers the wax phase to the skin, or in case of a wax phase containing active ingredients that the latter are delivered onto the skin.

The wax phase may further contain dye-precursors, i.e. agents that become dyed upon influence of physical or chemical factors. In particular embodiments the wax phase may contain dye-precursors that react with certain agents that are present in the aqueous phase so as to form a dye. Similarly, the dye-precursors may be present in the aqueous phase and become transferred into dyes upon interaction with certain chemicals incorporated into the wax phase.

The wax dispersion can also be formulated to or into beads or capsules. Particularly such beads are polymeric beads wherein the wax dispersion is entrapped in whatever form. The terms 'beads' or 'polymeric beads' are meant to comprise any form of discrete, free-flowing powders, beads or capsules which envelope, coat or contain a wax dispersion in a mono- or polymeric matrix or capsule. These terms are also meant to include porous beads or 'microsponges' and 'microcapsules', the latter being beads of smaller size. The beads may be coated with a suitable coating material that protects the interior of the bead or controls the release of the wax dispersion entrapped therein Where the wax dispersion is viscous enough, e.g. by addition of sufficient amounts of thickener, the coating on the bead itself may contain a wax dispersion. In the latter instance, the coating is laid on an inert core or on a core containing wax dispersion and/or other ingredients.

Formulation of the wax dispersion in beads may be done for protecting the wax dispersion from external factors that may impact its integrity. However, it is costly done for allowing controlled release of the wax dispersion.

The size of the capsules or beads is selected such that they are sufficiently big to contain the dispersion, relatively larger sizes being preferred; e.g; in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized up to about 300 µm (average diameter) having a large inner surface. These, are obtained by polymerization of particular monomers. Wax dispersion material can be entrapped therein either during this polymerization process or afterwards.

The capsules or beads may optionally contain one or more suitable disintegrating agents, in particular those mentioned in this specification. Upon contact with the appropriate external factor, the disintegrating agents will cause the capsules to break open thus allowing release of the wax dispersion entrapped therein.

Release of the wax dispersion from the beads or capsules can be the result of the rupture of the coating or from the matrix. This may be the result of physical factors such as pressure, strain or by shearing forces upon use of the applicator product, e.g. by rubbing the product to the skin or to a surface. Release of the wax dispersion may be due to the semi-permeable or porous nature of the bead or its coating or due to external factors such as contact with liquid media that cause the wax dispersion to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with the aqueous phase when using the applicator product, cause the capsules to disintegrate.

The beads or capsules can be made according to methodologies generally known in the art, for example by emulsion polymerisation.

The beads or capsules may be applied to any portion of the applicator but preferably they are concentrated at the surface or in the upper surface portion of the applicator. This allows maximal transfer of the wax dispersion to the skin or to the surface to which the product is applied.

The beads or capsules can be applied to the applicator in dry form by dusting, sifting, spraying and the like methods. They can also be printed or roll-coated in the form of a suitable liquid or paste. They can also be mixed with a suitable liquid, which can be a solvent that is inert towards the beads, or water, or the aqueous phase, and sprayed onto the applicator.

The composition of the wax phase has a melting point or range of above 25 °C, preferably in the range of 30 to 45 °C, more preferably in the range of 32 to 40 °C.

The water content of the preferred compositions of the wax phase is low, e.g. lower than 10 %, preferably lower than 6 %, more preferably lower than 3 %. In particular, the preferred compositions will be water free.

The wax phase is a waxy composition comprising at least one oil or wax component selected from dialkyl(ene) ethers, dialkyl(ene) carbonates, dicarboxylic acids or hydroxy fatty alcohols or mixtures thereof, the total composition thereof having a waxy consistency. The waxy phase in this preferred embodiment may contain - depending on the required profile - further waxy lipids and oils. It is essential however, that the melting point of the wax phase is above above 25 °C.

In a particularly preferred embodiment the wax phase is a waxy composition comprising:
(a) at least one oil or wax component selected from dialkyl(ene) ethers, dialkyl(ene) carbonates, dicarboxylic acids or hydroxy fatty alcohols or a mixture thereof;
(b) an active ingredient.

Particular dialkyl(ene) ethers, dialkyl(ene) carbonates, dicarboxylic acids or hydroxy fatty alcohols for use in the wax phase are those mentioned hereinabove.

The said waxy composition liquefies above 25 °C and/or has a water content of less than 10 %, preferably less than 6 %, more preferably less than 3 %. In particular said preferred or further preferred waxy composition is water-free, and will be such that it is not decomposed by the aqueous phase. As used herein, water-free generally means that the phase is composed of materials of low water content to which no water has been added.

The wax phase may in particular contain further waxy lipid components or oils.

The wax phase can also contain liquid diakl(ene) ethers, dialkyl(ene) carbonates, dicarbonic acids or hydroxy fatty alcohols, however in such amounts that the melting point or range of the total composition of the wax phase does exceed 25 °C, and more preferably is within the temperature ranges mentioned above.

The preferred wax compositions may contain further ingredients, in particular any ingredients having a waxy consistency, e.g. at concentrations of less than 20 %.

In further preferred embodiments, the wax phase in the wax dispersions of the invention has a melting point which is in the range of 35 - 50 ° C, in particular of 35 - 45 ° C and more in particular of 37 - 42 ° C. These are preferred conditions for allowing the wax phase, when applied to the applicator, to remain finely dispersed and only upon application at body temperature to become liquid. Applicators that have been treated with such wax dispersions are particularly stable during storage and mixing of the phases is avoided.

In particularly preferred embodiments, the products of this invention have a wax dispersion that comprises:
(a) 1 *-* 50 weight % of a wax phase comprising:
   (a1) 0.1 - 30 weight % of at least an oil or wax component selected from C₁₄-C₃₀-dialkyl ethers, C₁₄-C₃₀-dialkyl carbonates, C₄-C₃₄-dicarbonic acids or C₁₂-C₃₀-hydroxyfatty alcohols or any mixture thereof ;
   (a2) 0.1 - 10 % (w/w) of at least one oil;
   (a3) 0.1 - 10 % (w/w) of at least one non-ionic emulsifier
   (a4) 0.1- 40 % (w/w) of at least one further waxy lipid component; w/w relative to the total weight of the wax dispersion;
(b) 50 - 99 % (w/w) of an aqueous phase; w/w relative to the total weight of the wax dispersion.

### Polymers

In further embodiments of the invention, the wax dispersion contains at least one polymer, preferably in the aqueous phase. The presence of polymers helps to improve the ability to form the fine wax particles in the wax dispersions of the invention. The polymers are usually present in quantities ranging from 0.01 - 5 w/w-%, in particular from 0.05 - 3 and more in particular from 0.1 - 2 w/w-% relative to the total weight of the wax dispersion.

Anionic, zwitterionic, amphoteric and nonionic polymers that can be used are, for example, vinylacetate/crotonic acid-copolymers, vinylpyrrolidon/vinylacrylate-copolymers, vinylacetate/butylmaleate/ isobornylacrylate-copolymers, methylvinylether/maleic acid anhydride-copolymers and their esters, polyacrylic acids which are either non cross-linked or are cross-linked with polyoles, acrylamidopropyltrimethylammonium chloride/·acrylate-copolymers, octylacrylamide/methylmethacrylate/tert.butylaminoethylmethacrylate/2-hydroxypropylmethacrylate-copolymers, polyvinylpyrrolidone, vinylpyrrolidone/vinylacetate-copolymers, vinylpyrrolidone/dimethylaminoethylmethacrylate/vinyl caprolactam-terpolymers as well as optionally derivatized cellulose ethers and silicones.

Of particular interest are polysaccharides, in particular xanthan gum, guar-guar, agar-agar, alginate and tyloses, carboxymethylcellulose and hydroxyethylcellulose, polyacrylates (e.g. Carbopole^{®} from Noveon or Synthalene^{®} from 3V/Sigma), polyacrylamides, polyvinylalcohol and furthermore higher molecular polyethylene glycolmono- and -di-ester of fatty acids.

Appropriate cationic polymers are for example cationic cellulose derivatives, e.g. quaternized hydroxyethyl cellulose (commercialized under the trade name Polymer JR 400^{®} by Amerchol), cationic starches, copolymers of diallylammonium salts and acrylamides, quaternized vinylpyrrolidone/vinylimidazole-polymers (for example Luviquat^{®} of BASF), condensation products of polyglycols and amines, quaternized collagen polypeptides, such as, for example, lauryldimonium hydroxypropyl hydrolyzed collagen (Lamequat^{®}L/Grünau), quaternized wheat polypeptides, polyethylene imines, cationic silicone polymers, e.g.. amodimethicone, copolymers of adipinic acid and dimethylaminohydroxypropyldiethylenetriamine (Cartaretine^{®}/Sandoz), copolymers of acryl acid with dimethyldiallylammonium chloride (Merquat^{®} 550/Chemviron), polyaminopolyamides, cationic chitine derivatives such as, for example, quaternized chitosans, optionally dispersed in microcrystalline form, condensation products derived from dihalogenalkylenes, such as, for example dibromobutane with bis-dialkylamines, e.g. bis-dimethylamino-1,3-propane, cationic guar-gum, such as, for example, Jaguar^{®} CBS, Jaguar^{®} C-17, Jaguar^{®} C-16 from Celanese, quaternized ammonium salt-polymers, e.g. Mirapol^{®} A-15, Mirapol^{®} AD-1, Mirapol^{®} AZ-1 from Miranol.

Preferred are water-soluble or water-swellable polymers, in particular non-ionic and anionic polymers. Particularly preferred are polymers selected from the group of polyacrylates, polysaccharides polyacrylamides or any mixture of these polymers.

### Emulsifiers

The stability of the fine-particulate wax dispersions of the invention is increased by adding suitable emulsifiers. These in particular are ionic and non-ionic emulsifiers, the latter being preferred. The emulsifiers can be of the W/O (preferably for use in the wax phase) or the O/W (preferably for use in the aqueous phase) type. The addition of an emulsifier allows the incorporation of hydrophilic components or agents, or also of small amounts of water into the wax phase, and vice versa of lipophilic components or agents into the aqueous phase.

Preferred are non-ionic emulsifiers, which typically have good skin compatibility and mildness and are environmentally friendly. They moreover add to the stability of the fine-particulate dispersions. Of particular interest are combinations of W/O and O/W-emulsifiers which yield dispersions of increased stability and improved sensorial properties.

The wax dispersions contain the emulsifier(s) in quantities which are in the range of 0.1 to 10 w/w%, in particular of 0.5 to 7 w/w% and more in particular of 1 to 5 w/w% relative to the total weight of the wax dispersion.

### Non-ionic emulsifiers

The following are suitable non-ionic emulsifiers:
(1) Addition products of 2 to 50 moles of ethylene oxide and/or 1 to 20 moles propylene oxide to linear fatty alcohols having 8 to 40 C-atoms; to fatty acids with 12 to 40 C-atoms and to alkylphenols with 8 to 15 C-atoms in the alkyl rest.
(2) C₁₂₋₁₈-fatty acid mono- and -diesters of addition products of 1 to 50 moles of ethylene oxide to glycerine.
(3) Addition products of ethylene oxide to glycerine mono- and -diesters; and sorbitan "mono- and -di-esters of saturated and unsaturated fatty acids with 6 to 22 C-atoms and their ethylene oxide addition products.
(4) Alkyl mono- and -oligoglycosides with 8 to 22 C-atoms in the alkyl rest.and their ethoxylated analogues.
(5) Addition products of 7 to 60 moles of ethylene oxide to castor oil and/or hardened castor oil.
(6) Polyol- and in particular polyglycerine esters, such as e.g. polyol poly-12-hydroxystearate, polyglycerine polyricinoleate, polyglycerine diisostearate or polyglycerine dimerate; also applicable are mixtures of components of several of these substance classes.
(7) Addition products of 2 to 15 moles of ethylene oxide to castor oil and/or hardened castor oil.
(8) Partial esters derived from linear, branch chained, unsaturated or saturated C₆-₂₂-fatty acids, ricinoleic acid as well as 12-hydroxystearic acid and glycerine, polyglycerine, pentaerythrite, dipentaerythrite, sugar alcohols (e.g. sorbitol), alkylglucosides (e.g. methylglucoside, butylglucoside, laurylglucoside) as well as polyglucosides (e.g. cellulose), or mixed esters such as e,g. glyceryl stearate/citrate and glyceryl stearate/lactate.
(9) Polysiloxane-polyalkyl-polyether-copolymers and the respective derivatives thereof.
(10) Mixed esters from entaerythrite, fatty acids, citric acid and.fatty alcohols and/or mixed esters of fatty acids with 6 to 22 C-atoms with methylglucose and polyoles, respectively glycerine or polyglycerine.
(11) Polyalkylene glycols.

The addition products of ethylene oxide and/or of propylene oxide and fatty alcohols, fatty acids, alkylphenols, glycerine mono- and -di-esters as well as sorbitan mono- and -diesters of fatty acids or of castor oil are known and commercially available products. Usually these are mixtures of homologues of which the average degree of alkoxylation corresponds to the ratio of starting quantities of ethylene oxide and/or propylene oxide and substrate, with which the addition reaction is conducted. Depending upon the degree of alkoxylation these products are either W/O- or O/W-emulsifiers. C₁₂₋₁₈-fatty acid mono- and -di-esters of addition products of ethylene oxide to glycerine are known as re-fatting agents in cosmetic applications.

Of particular interest are the non-ionic emulsifiers of group (4) in the.above list. Non-ionic emulsifiers from the group of alkyloligoglucosides and in particular the C₈-C₂₂-alkylmono- and -oligoglucosides, are attractive due to their good skin-compatibility. Alkyl monoglucosides, having a sugar rest linked to an alkyl group, as well as alkyl oligomeric glucosides having a degree of oligomerisation of up to about 8, or mixtures thereof can conveniently be used. An example is a C₈-C₁₆-alkylglucoside with a degree of oligomerisation of between 1 - 2 which is commercially available under the tradename Plantacare^{®}. Further suitable non-ionic emulsifiers are the acylglucamides.

Particular useful and mild emulsifiers are polyolpoly-12-hydroxystearates and mixtures thereof with other components, that are available under the tradename

"Dehymuls^{®} PGPH" (W/O-emulsifier) or "Eumulgin^{®} VL 75" (1:1 w/w mixture with coco-glucosides, O/W-emulsifier) or Dehymuls^{®} SBL (W/O-Emulsifier) from Cognis Deutschland GmbH. The polyol components of these emulsifiers can be derived from materials that have at least two and in particular 3 to 12 and more in particular 3 to 8 hydroxyl groups, and 2 to 12 carbon atoms.

Lipophilic W/O-emulsifiers in principle are emulsifiers with a HLB-value in the range of 1 to 8, that are described, for example, in Kirk-Othmer, "Encyclopedia of Chemical Technology", 3rd Ed., 1979, Vol. 8, p. 913. The HLB-value of ethoxylated products is calculated by the formula: HLB = (100 - L) : 5, wherein L is the percentage (in weight %) of lipophilic groups, i.e. of fatty alkyl- or fatty acyl groups in the ethylene oxide adducts.

Particularly attractive W/O-emulsifiers are the partial esters of polyoles, in particular of mono-, di-, tri-, or sesqui esters of fatty acids of polyoles, more in particular of C₃-₆-polyoles, such as, for example, glyceryl monoesters, partial esters of pentaerythrite or carbohydrate esters, in particular the sorbitan mono-, di-, tri- or sesqui fatty esters, in particular the sorbitan stearates, oleates, erucates, ricinoleates, hydroxystearates, isostearates, tartrates, citrates, and maleates. Also of interest are addition products of ethylene oxide to these sorbitan esters, in particular with 1 to 30, respectively 5 to 10 moles of ethylene oxide.

In case it is desirable to, incorporate water-soluble active ingredients and/or small amounts of water into the wax phase it may be advantageous to add an emulsifier selected from the group of non-ionic O/W-emulsifiers (HLB-value: 8 - 18) and/or solubilizers. These can for example be the already mentioned ethylene oxide-adducts with a corresponding high degree of ethoxylation e.g. 10 - 20 ethylene oxide units in the case of O/W-emulsifiers and 20 - 40 ethylene oxide units for so-called solubilizers. Particularly attractive as O/W emulsifiers are Ceteareth-12 and PEG-20 stearate. Particularly attractive solubilizers are Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycol ether) and Eumulgin^{®} SML 20 (INCI: Polysorbate-20).

Non-ionic emulsifiers of the group of alkyl-oligoglycosides are particularly skin-compatible and therefore preferred as O/W-Emulsifiers. C₈-C₂₂-alkyl mono- and-oligoglycosides, their preparation and use have been described in the prior art. Oligoglycosides are meant to comprise oligomeric glycosides with a degree of oligomerisation of up to about 8. The degree of oligomerisation can also be a statistical average used for those products comprised of a specific range of oligoglycosides. An example is the product sold under the tradename Plantacare^{®}, which has a C₈-C₁₆-alkyl group glycosidically bound to an oligoglucoside rest, having an average degree of oligomerisation between 1 and 2.

Other non-ionic emulsifiers are the acyl glucamides. Preferred is the product sold under the tradename Emulgade^{®} PL 68/50 (Cognis Deutschland GmbH) which is a 1:1-mixture of alkyl polyglucosides and'fatty alcohols, and a mixture of lauryl glucoside, polyglyceryl-2-dipolyhydroxystearate, glycerine and water, sold under the trade name Eumulgin^{®} VL 75.

### Additional ingredients for either one or both phases in the wax dispersions

The wax dispersions of the invention may contain further ingredients that may be present in the wax phase or in the aqueous phase that carries the dispersed particles or in both. Examples are active ingredients, moisturizers, anti-perspirants, UV-absorbing agents, etc.

### Active ingredients

The wax dispersion may contain active ingredients for application to the skin. The wax phase in the wax dispersions preferably contains oil-soluble or hydrophobic active agents, while the aqueous phase preferably contains water-soluble or hydrophilic active agents. However by using suitable emulsifiers oil-soluble or lipophilic active ingredients can be incorporated into the aqueous phase and vice versa, water-soluble or hydrophilic agents can be incorporated in the wax phase of the wax dispersions.

The active ingredients, which may be lipophilic or hydrophilic, can be mixed with or incorporate into suitable carriers. These comprise any skin-acceptable inert materials that are known for formulating active ingredients. The carriers can.be finely or more coarsely divided powders, or even granulates. They can comprise starches, sugars, binders, lubricants, diluents, fillers, disintegrating agents, granulating agents and the like components. The nature of the carrier materials will depend on the active ingredient that is formulated therein and on the type of formulation that is desired.

Particular carriers for incorporating active ingredients are beads wherein the active ingredient is entrapped in some form. The terms 'beads' or 'polymeric beads' are meant to comprise any form of discrete, free-flowing powders, beads or capsules which envelope, coat or contain an active ingredient in a mono- or polymeric matrix or capsule. These terms are also meant to include porous beads or 'microsponges' and 'microcapsules', the latter being beads of smaller size. The beads may be coated with a suitable coating material that protects the interior of the bead or controls the release of the active ingredient entrapped therein. The coating on the bead itself may contain the active ingredient in which case the coating is laid on an inert core.

Formulating an active ingredient in beads can be for protecting the active from environmental factors but is mostly done for allowing controlled release of the active.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average sizes in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized from about 5 to about 300 µm (average diameter) having a large inner surface. These are obtained by polymerization of particular monomers. An active ingredient can be entrapped therein either during this polymerization process or afterwards. Microsponge-based carriers may be used to protect the active ingredient entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the active ingredient entrapped therein.

The capsules can be incorporated into the wax particles or, which is preferred, in the aqueous phase, or into both. They can also be applied to the applicator prior to the introduction of the lipid and aqueous phase. They can even be introduced during the manufacturing process of the applicator itself.

Release of the active from the beads or capsules can be the result of the rupture of the coating or the matrix. This may be the result of physical factors such as pressure, strain or by shearing forces upon use of the applicator product, e.g. by rubbing the product to the skin or to a surface. Release of the active ingredient may be due to the semipermeable or porous nature of the bead or its coating or due to external factors such as contact with liquid media that cause the active ingredient to become extracted; or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with the aqueous phase when using the applicator, cause the capsules to disintegrate.

The beads or capsules can be made according to methodologies .generally known in the art, for example by emulsion polymerization.

The beads or capsules may be applied to any portion of the applicator but preferably they are concentrated at the surface or in the upper surface portion of the applicator. This allows maximal transfer of the active ingredient to the skin or to the surface to which the product is applied.

The beads or capsules can be applied to the applicator in dry form by dusting, sifting, spraying and the like methods. They can also be printed or roll-coated in the form of a suitable liquid or paste. They can also be mixed with a suitable liquid, which can be a solvent that is inert towards the beads, or water, or an aqueous phase, and sprayed onto the applicator.

Examples of active agents which may be hydrophobic or hydrophilic for use in the products of the invention compounds that have a cosmetic or therapeutic effect on the skin, hair, or nails, e.g., lightening agents, darkening agents such as self-tanning agents, anti- acne agents, shine control agents, anti-microbial agents, anti- inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

Examples of active ingredients are hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers,;spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides such as those disclosed in WO-00/15188, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera and soy, and derivatives and mixtures thereof. Of particular interest are active agents that can be used for treating skin that shows inflammatory reactions, that is irritated, red or damaged. Examples of such agents are zinc compounds or sulphur. Examples of vitamins include, but are not limited to, vitamin A, vitamin Bs such as vitamin B3, vitamin B5, and vitamin B12, vitamin C, vitamin K, and vitamin E and derivatives thereof.

The cosmetically active agent will typically be present in the wax dispersions in an amount of from about 0.001% to about 10% by weight of the dispersion, e.g., about 0.01% to about 10% such as about 0.1% to about 5%.

Typical examples of anti-microbial agents are those active against gram-positive bacteria such as 2,4,4'-trichloro-2'-hydroxydiphenyl ether, chlorohexidine (1,6-di-(4-chlorophenyl-biguanido)hexan) or TCC (3,4,4'-trichlorocarbanilide). Furthermore many odorants and etheric oils have anti-microbial activity. Also glycerine monolaurate, glycerine stearate, glycerine oleate as well as glycerine dioleate have been found to possess anti-microbial activity and are particularly attractive for use in products that are applied on babies because of their mildness and lack of side effect. The quantity of anti-microbial agents can vary but usually is in the range of about 0.1 to 2 % (w/w) - relative to the total amount of the wax dispersion.

### Further additional ingredients

The wax dispersions can contain further ingredients such as moisturizers, refattening agents, thickeners, powders, biogenic actives, deodorants, film formers, UV sunscreen filters, anti-oxidants, hydrotropes, preservatives, insect repellents, self tanners, solubilizers, perfumes, dyes, pigments, and the like.

Preferred anti-oxidants are carotinoids; carotines (e.g.. α-carotine, β-carotine, lycopine)and derivatives thereof, α-hydroxyacids (e.g. citric acid, lactic acid, malic acid), EDTA, vitamin C and derivatives (e.g. ascorbyl palmitate, Mg-ascorbyl phosphate, ascorbyl acetate), tocopherole and derivatives (e.g. vitamin-E-acetate), butylhydroxy toluol and butylhydroxy anisol.

Biogenic active ingredients are for example tocopherol, tocopherol acetate, tocopherol palmitate, ascorbic acid, desoxyribonucleinic acid, retinol; bisabolol, allantoin, phytantriol, panthenol, α-hydroxycarbonic acids, amino acids, ceramides, pseudoceramides, essential oils, extracts and vitamin complexes.

### Moisturizers

The wax dispersions, either in the wax phase or in the aqueous phase, may further contain one or more moisturizers. These are added to improve the sensoric properties as well as to regulate skin hydration. These agents additionally can improve the penetration of the composition in or into the applicator.

Moisturizers typically are present in quantities of 1-20 % (w/w), preferably of 5-15 % (w/w), and more preferably 5 -10 % (w/w) relative to the total amount of the wax dispersion.

Suitable moisturizers are a.o. amino acids, pyrrolidone carbonic acid, lactic acid and its salts, lactitol, urea and urea derivatives, ureic acid, glucosamine, creatinine, hydrolysis products of collagen, chitosan or chitosan salts/-derivatives, and in particular polyols and polyol derivatives (e.g. ethylene glycol, propylene, glycol, butylene glycol, pentylene glycol, hexylene glycol, erythrite, 1,2,6-hexanetriol, polyethylene glycols such as PEG-4, PEG-6, PEG-7, PEG-8, PEG-9, PEG-10, PEG-12, PEG-14, PEG-16, PEG-18, PEG-20, PEG-135, PEG 150), sugar and sugar derivatives (a.o. fructose, glucose, maltose, maltitol, mannite, inosite, sorbite, sorbityl silandiol, sucrose, trehalose, xylose, xylit, glucuronic acid and its salts), ethoxylated sorbitol (Sorbeth-6, Sorbeth-20, Sorbeth-30, Sorbeth-40), honey and hydrogenated honey, hydrogenated starch hydrolysates, as well as mixtures of hydrogenated wheat protein, hydrolyzed milk protein, lecithin, pythantriol, hyaluronic acid and salts thereof, and PEG-20-acetate copolymers. Particularly preferred moisturizers are glycerine, diglycerine and triglycerine.

The products according to this invention can be used as anti-perspirants or deodorants, in particular as wipes or tissues for use in these applications. In products for these applications either the wax dispersion or, if present, the additional aqueous phases, contain actives that have deodorizing and/or anti-perspirant properties. Actives that can be used to this purpose are anti-perspirant agents such as, for example, aluminium chlorohydrate, aluminium-zirconium-chlorohydrate as well as zinc salts.

The products according to the invention can also be used in sunscreen applications and in that instance take the form of sunscreen wipes. In these products the wax particles and/or aqueous phase contains one or more sunscreen filters which are for example organic substances that are capable of absorbing ultraviolet radiation and to set free the absorbed energy as longer-wave radiation, e.g. as thermic energy. UVB-filters can be oil or water-soluble.

Apart from the above-mentioned soluble substances, there can also be used insoluble sunscreen pigments, namely finely dispersed metal oxides or metal salts.

### Preparation of-the Wax Dispersion

The wax dispersions can be obtained by preparing a pre-emulsion containing the wax components in an aqueous phase at a temperature above the melting point or melting range of the wax phase (usually above 40 - 90°C) and introducing this pre-emulsion while being at the previously mentioned temperature into a cold aqueous phase (usually at a temperature in the range of 1 - 30 °C) that contains the polymeric components. The temperature of the cold aqueous phase preferably is lower or equal to 25°C, particularly preferred is a temperature of 5 - 25 °C. During the addition of the 'warmer' pre-emulsion the temperature of this cold aqueous phase should be kept such that it does not reach the melting point or range of the wax phase, and consequently cooling may be required. The wax components can be selected among any of the above-mentioned wax components.

The polymeric components preferably are water-soluble or water swellable. By using polymers very fine particle and stable wax dispersions are obtained, which even do not separate over long periods of time. Preferably, the pre-emulsion contains polymer as well. Appropriate polymers have been described before. In a preferred execution of the process the polymer is selected from the group of polyacrylates, polysaccharides, polyacrylamides or any mixture of these polymers.

The incorporation of the hot preemulsion into the cold water phase can be done by applying known methods. To obtain finely dispersed particles it is preferred to homogenize the preemulsion at least once before adding it to the water phase. In a further preferred embodiment the preemulsion is cooled using a heat exchanger before addition to the water phase, preferred to temperatures below 50°C. Further preferred is to spray the preemulsion under pressure through a nozzle into the water phase. The set up for the exact pressure conditions is dependent on the used equipment.

In a further preferred execution of the process, the aqueous phase contains an oil or wax component selected from the group of dialkyl(ene) ethers, dialkyl(ene) carbonates, dicarboxylic acids or hydroxyfatty alcohols or any mixture thereof and at least one emulsifier. Particular such components have been mentioned herein.

Preferably the quantities of the starting materials are selected such that wax dispersions are obtained that comprise (a) 1 - 75 w/w-% of a wax phase and (b) 25 - 99 w/w-% of a water phase, w/w.relative to the total weight of the wax dispersion.

### Application of the wax dispersion

The wax dispersion may be applied to the applicator in various ways. Preferably the wax dispersion is applied at the surface or at the surface portion of the applicator, on one or on several side.

The wax dispersion can be applied evenly or non-evenly to the applicator, non-evenly meaning that the distribution of the amount of the wax dispersion varies over the area of the applicator, i.e. some areas of the applicator can have greater or lesser amounts of the wax dispersion. Preferably the wax dispersion is evenly applied to the area of the applicator.

The wax dispersion can be applied discontinuously or continuously to one or several sides of the applicator, or it may even be applied as a complete covering of one or several surfaces of the applicator.

The wax dispersion preferably is applied in a discontinuous pattern, to one or several sides of the applicator. To this purpose the wax dispersion is applied in a predetermined, controlled manner to specific areas of the applicator. A discontinuous pattern is one in which the wax dispersion has been applied to distinct regions separated by regions of the applicator which are free of the wax dispersion. The wax dispersion in that instance is applied to defined parts or regions of the applicator which may take a variety of forms. The wax dispersion may in particular be applied as described above. Particular forms in which the wax dispersion may be applied are, e.g. stripes, dots or spots, geometric configurations, either of regular or irregular shape, for example circles, ellipses, squares, rectangles and the like, logos, text, letters or any other non-continuous pattern, including the patterns described hereinabove more generally for the application of the lipid and aqueous phase.

Discontinuous patterns also comprise essentially networks of larger patterns of the wax dispersion. In a preferred embodiment, the wax dispersion is present as discrete stripes which can be disposed discontinuously, i.e. interrupted, or preferably continuous over the whole surface of the applicator. The stripes may also form a pattern of discrete segments which collectively comprise a stripe or they may have a repetitive pattern such as a sinusoidal shape or wave-like and the like pattern. If waving stripes are selected, preferably the stripes are in phase; so that parallelism is maintained and each stripe remains equally spaced from the adjacent stripes.

The stripes are preferably oriented in the machine direction, for ease of manufacture.

In another embodiment more than one wax dispersion may be applied to one or several sides of the applicator. For example one wax dispersion may be applied on the entire surface or part of the surface of one side of the applicator, whereas another wax dispersion is applied on the entire other side or only partly; either with the same or another pattern than the other wax dispersion. Particular such embodiments are those having two different wax dispersions on the same side e.g. in parallel stripes or other patterns with the same or different colors.

In a particular embodiment, not more than half of the surface of the applicator, either on one side or, which is preferred, on several sides is carrying or covered by the wax dispersion. In a preferred embodiment, the wax dispersion is present at the surface on several sides, covering not more than 50 % of the applicator's surface, in particular covering not more than 35 % or not more than 25 % of the surface. In a particularly preferred embodiment, the wax dispersion is present as stripes, in particular as parallel stripes running in parallel with the side of the applicator, covering not more than half or, more in particular 25 % of the surface. In another particularly preferred embodiment, the wax dispersion is present as dots, equally spread over the entire surface of the applicator, covering not more than 50 % of the surface.

There can be embodiments with more or less regularly shaped dots, other embodiments have circle-shaped dots, others have ellipsoids, while still others have mixed patterns, e.g. combinations of circles and ellipsoids, of regularly shaped dots and circles and the like.

In case of stripes, the width thereof preferably is between 1 to 10 mm, more preferably between 3 to 7 mm. In case of dots, round shapes are preferred, e.g. circles or ellipsoids, with an average diameter between 1 to 10 mm, more preferably between 3 to 7 mm. There can be stripes with different widths on one product, and there can be dots of different size on one product. An example of an embodiment of the latter is an applicator with circles of a certain size and ellipses of a different size, or of circles with different sizes.

The wax dispersion may be colorless or colored, i.e. mono- or multi-colored. Multi-colored patterns are obtained by applying several wax dispersions that have been dyed differently. A colored wax dispersion will alert the user of the fact that the applicator is covered by a special material that contains an active ingredient or it may also make the product aesthetically attractive.

In another embodiment the applicator itself is colored, either at several sides or at one side, over the complete surface or only at parts. If the color is present only at parts of the applicator it preferably will take the shapes and forms described in connection with the patterns that the wax dispersion may take. In another embodiment only the space between the surface portions at which the wax dispersion is applied is colored thus leaving the areas of the wax dispersion uncolored. In this way, the patterns of the wax dispersion will appear as uncolored patterns.

A preferred pattern for coloring the applicator is in stripes, in particular stripes oriented in the machine direction. Examples of such embodiments are those wherein the colored stripes or the area between the colored stripes are covered with wax dispersion. In the former instance the wax dispersion stripes are colored, in the latter they are uncolored.

The wax dispersion, which itself can be colored or uncolored, may be applied-to the colored applicator in a number of different ways.

In case of applicators having a completely colored surface, the wax dispersion can be applied over the whole surface thus resulting in a different or altered color, e.g. a more pale color where the wax dispersion is white or opaque. The wax dispersion can also be applied in certain patterns, thus resulting in multicolored products or where the wax dispersion is white or opaque in products with mono-colored patterns. Also in this instance, the preferred pattern is in stripes.

In still a further embodiment, the applicator is colored in certain patterns and the wax dispersion is applied on these patterns or part of these patterns. Also in this instance the wax dispersion may be colored or uncolored, i.e. white, opaque or transparent. In case the wax dispersion is white or opaque its thickness may be selected such that the color of the underlying section of the applicator is visible thus giving the consumer the impression that a wax dispersion containing a particular ingredient is present.

The wax dispersion is typically applied in an amount of about 3 to 200 g/m² preferably in an amount of about 3 to 40 g/m², preferably from about 10 to about 20 g/m², either on one side or, preferably, on several sides of the applicator. Or, alternatively, the wax dispersion is applied in an amount of about 0.06 g to 0.8 g per gram of substrate, preferably from about 0.20 g to 0.40 g per gram of dry substrate.

The wax dispersion can be applied to the applicator by any method that can be used to contact or impregnate a liquid or semi-liquid material to or in an applicator. The wax dispersion may be applied by bathing the applicator into the wax dispersion. Where the latter is semi-solid, it can be made more liquid by dilution with water or an aqueous phase which may be evaporated afterwards.

The wax dispersion can also be applied by any method that allows coating of the lipid material onto the surface of the applicator. As used herein the term 'coating' refers to printing, covering, overlaying, finishing, spraying, extruding, laminating or any other method of applying the phase to the surface of the applicator.

A particular coating technique is extrusion wherein the composition is forced through tubes in contact with the applicator while the applicator passes across the tube. A preferred technique comprises contacting the applicators with a head optionally equipped with a slit blade, i.e. a blade having cut-out areas, wherefrom the wax dispersion is extruded. By this technique the pattern, e.g. stripes, is defined by the design of the blade, which is inserted in the application head. The cut-out areas define the width of the stripes. Another application technique involves spraying or drippling the composition on a rotating surface such as calender roll that then transfers the composition to the surface of the substrate.

Still another technique is based on traditional printing technologies which comprise, for example, screen printing, roller printing and gravure printing. In general, printing comprises techniques wherein a rotating surface is provided with elevations (by engraving, embossing or similar techniques) and the elevations are contacted with the wax dispersion, e.g. by running it through a bath with wax dispersion, and thus printed on the applicator. Whereas the slot coating technique preferably leads to patterns in machine direction as e.g. stripes, roller printing, screen printing or "Flexo coat" printing can be used to get a cross direction pattern.

Another technique to apply the wax dispersion is by using a screen printing procedure where the wax dispersion is introduced into a rotating roll and squeezed through a metal screen which covers the roll. This leads, depending on the design of the screen, to a defined pattern on the applicator like stripes, dots, squares, circles and the like, or even logos and text.

The wax dispersion may also be applied as beads as described above. The wax dispersion can also be entrapped into dots which are small bubbles of polymeric or other material that contain wax dispersion. In this instance it may be advantageous to make the wax dispersion more viscous. The dots may be introduced on the applicators by techniques similar as for applying stripes or other patterns, e.g. by contacting the sheet with a roll whereon the dots have been applied or a similar technique.

The wax dispersion preferably is applied such that it is present at the surface of the applicator because of its physical location in that instance, the wax dispersion is readily available to be spread onto the skin during usage. As a result, the effectiveness with which the wax dispersion is transferred to the skin during use, the availability and therefore the effectiveness of active ingredients is increased compared to products where the active agent is simply incorporated into a single continuously applied phase

### Additional aqueous phases

The products of the invention may contain, apart from one or more wax dispersion, one or more additional aqueous phases. These additional aqueous phases can be any of the art-known aqueous based formulations used to impregnate wipes. Beside water the additional aqueous phases may also contain further ingredients or additives such as surfactants, emulsifiers, consistency factors, conditioners, moisturizers, thickeners, preservatives, deodorants, film formers, powders, sunscreen filters, anti-oxidants, hydrotropes, active ingredients, in particular dermatologically active ingredients, fragrances and the like. Active ingredients as mentioned herein comprise, for example, anti-inflammatories, anti-bacterials, anti-fungals, biogenic actives and the like agents. Active ingredients suited for topical applications are particularly preferred.

The additional aqueous phases may contain suitable dyes that preferably are hydrophilic. In one type of embodiments, the wax dispersion is applied discontinuously as a layer e.g. in the form of stripes leaving areas with only aqueous phase, which areas are colored. This allows the manufacture of applicators with colored patterns, e.g. colored lines or even multicolored patterns when the wax dispersion itself is also colored.

The additional aqueous phases may further contain lipophilic dyes; which upon contact with the wax dispersion migrate into that phase and cause it to become colored.

The additional aqueous phases may further contain one or more preservatives.

Suitable surfactants for the additional aqueous phases comprise:
alkyl sulfates, e.g. sodium lauryl sulfate, ammonium lauryl sulfate, sodium cetearyl sulfate;
alkyl sulfoacetates, e.g. sodium lauryl sulfoacetate;
alkyl ether sulfates e.g. sodium laureth sulfate, sodium trideceth sulfate; sodium oleth sulfate, ammonium laureth sulfate;
alkyl ether sulfosuccinates, e.g. disodium laureth sulfosuccinate;
alkyl glycosides, e.g. decyl glucoside, lauryl glucoside;
alkyl isothionates;
amphoterics, e.g. cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium salts of the aforementioned amphoterics, capryl/capramidopropyl betaine, undecyleneamidopropyl betaine, lauramidopropyl betaine and fatty alcohol polyglycol ethers.

Suitable conditioners are e.g. alkylamido ammonium lactate, cetrimonium chloride and distearoylethyl hydroxyethylmonium methosulfate and cetearyl alcohol, cetyl dimethicone, cetyl ricinoleate, dimethicone, laureth-23, laureth-4, polydecene, retinyl palmitate, agents selected from glyceryl monooleate and cocoglucoside including mixtures thereof (in particular the product 'Lamesoft**^{®}**' of Cognis which is a mixture of these two components), quaternized protein hydrolysates, quaternized cellulose and starch derivatives, quaternized copolymers of acrylic or methacrylic acid or salts, quaternized silicone derivatives, silicone oils, cyclomethicones, and the like agents, including mixtures thereof.

Suitable thickeners are e.g. acrylates/steareth-20 methacrylate copolymer, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl.silylate, xanthan gum, hydrogenated butylene/ethylene/styrene copolymer.

The additional aqueous phases may further comprise film-forming substances like chitosan and derivatives thereof, derivatives of poly acrylic acid, polyvinyl pyrrolidone and its derivatives, and the like.

The additional aqueous phases may contain pH sensitive components, i.e. components that change properties upon change of pH. The change of pH may occur when contacting the applicator with the skin whereupon the pH changes from the pH of the product which usually is about pH 7 to the skin pH which is about pH 5.5. pH sensitive agents for example comprise particular emulsifiers, stabilizers, surfactants viscosity regulating agents, chelators and the like.

In one embodiment an appropriate emulsifier is selected that is pH sensitive in this pH range in that it changes its emulsifying capacity, preferably increases its emulsifying capacity, so that upon contact with the skin an emulsification process occurs causing an interaction between the aqueous and the wax phase in the wax dispersion, or if present between any additional aqueous phase and the wax phase in the wax dispersion.

The aforementioned change of pH that occurs upon application of the product to the skin may also promote the release from active ingredients, in particular actives that are pH sensitive, e.g. actives having a pH dependent solubility.

### Application of the additional aqueous phases

The additional aqueous phases may be applied to the applicator using methods generally known in the art for applying aqueous liquid lotions such as spraying, dripping, immersing and the like techniques. A preferred application method for the additional aqueous phases is by spraying with a suitable nozzle or by drippling, for example by using a perforated tube having holes or slits. The immersing technique can be done by running the applicators through a bath holding the additional aqueous phases and subsequently controlling the amount of liquid that is absorbed by pressing.

The additional aqueous phases may be applied in various ways as described for the wax dispersion, evenly or non-evenly, continuously or non continuously, at-the surface or surface portion or, preferably; throughout the whole of the applicator. Optionally some parts of the applicator can be left dry, i.e. not having the lipid and the additional aqueous phases, or some parts can only have the lipid or the additional aqueous phases The additional aqueous phases may be applied at several sides or only at one side of the applicator.

The additional aqueous phases can be applied in various amounts, for example in an amount from about 0.1 g to about 10 g per gram of substrate and is typically applied in an amount from about 1.0 g to about 10 g per gram of substrate, preferably from about 2.0 g to about 5 g per gram of substrate, most preferably from about 2 g to about 4.5 g per gram of dry substrate, most preferably about 3.7 to about 3.8 g per gram substrate. Or, the additional aqueous phases is applied in an amount of about 4 to about 8 g per wipe sized 17.2 x 21 cm, most preferably about 6 g per wipe.. Or, alternatively, the additional aqueous phases are applied in an amount of about 3 to about 200 g/m², preferably of about 3 to about 40 9/m², more preferably from about 10 to about 20 g/m², either on one side or, preferably, on more sides of the applicator.

It may also be advantageous to only apply the additional aqueous phases to only those areas (or that side) of the applicator that have (or has) not already been covered with the wax dispersion.

Since in many cases the product is used as a cleansing article it is useful to design the additional aqueous phases as cleanser. Soils that are most difficult to clean are either water insoluble and/or strongly adhere to the skin: Therefore the additional aqueous phases is formulated such that it is capable of taking up water-insoluble materials.

### Manufacture

This invention further concerns a process for preparing a product as specified herein, said process comprising contacting an applicator with a wax dispersion, and if desired with further wax dispersions, said wax dispersion or further wax dispersions being as described herein.

In another aspect, the invention concerns a process for preparing a product as specified herein, said process comprising contacting an applicator with a wax dispersion and an additional aqueous phase, both being as described herein, or if desired with further wax dispersions and/or with further additional aqueous phases. In another aspect, the invention concerns the process of contacting the applicator simultaneously or subsequently with the wax dispersion and an additional aqueous phases.

In a particular execution, the process comprises contacting the applicator with a wax dispersion and subsequently with an additional aqueous phase.

This invention further concerns a process for preparing a product as specified herein, said process comprising contacting an applicator with a wax dispersion and an additional aqueous phase as described herein, and drying the product. The process comprises contacting the applicator simultaneously or subsequently with the wax dispersion and the additional aqueous phase and conducting a drying step.

The drying step may be applied at any time during the process, but it should be after the application of the additional aqueous phase. Drying can be done by conventional methods, e.g. by the application of hot air, or by leading the applicator through an oven or over a heated or warmed transport roll.

In case a wax dispersion has been applied prior to drying, the temperature of the air should be such that the wax dispersion does not melt. Application of air of ambient temperature may be recommendable in that instance.

In a particular execution, the process comprises contacting the applicator with a wax dispersion and subsequently with an additional aqueous phase, whereafter the thus obtained product is dried.

In another particular execution, the process comprises contacting the applicator with an additional aqueous phase, subsequently drying the thus treated applicator, whereafter the thus dried applicator is contacted with a wax dispersion.

In another particular execution, the process comprises contacting the applicator with an additional aqueous phase, subsequently with a wax dispersion after which the applicator is dried.

The wax dispersion and additional aqueous phases can be applied to the applicator at any time during the manufacturing process of the applicator. Preferably the wax dispersion and/or additional aqueous phase can be applied to the applicator after finishing the manufacturing process of the applicator.

Applicators with different coating and/or impregnation can be combined in one packaging. For example there can be a pack of applicators with increasing or decreasing amounts of wax phase. Or colored or uncolored applicators can be alternated.

The thus obtained applicators can be packed individually or can be packed in a determined number, e.g. a number between 10 and 30 in a suitable package, for example a plastic wrap, box and the like.

Applicators with different coating and/or impregnation can be combined in one packaging. For example there can be a series of applicators with increasing or decreasing amounts of wax dispersion. Or colored or uncolored applicators can be alternated.

### Application and properties

The products according to the present invention advantageously result in an optimal release of any active ingredient(s) present in the wax dispersion, in particular when incorporated in the wax particles of the wax dispersion, onto the skin during use.

Optimal release of active ingredients can be achieved by using a wax dispersion in which the wax particles are composed of a wax phase having a melting point or melting range which is equal to or slightly exceeds body temperature. Without being bound to theory, it is believed that this results in a quicker melting of the wax particles causing a faster and more efficient transfer and release to the skin of the active materials.

Optimal release of active ingredients is also promoted by incorporating a suitable emulsifier to cause a local emulsification process :on the skin during use of the applicators. Preferably the emulsifier is present in the aqueous phase of the wax dispersion, although, where additional aqueous phase is present, this phase may also contain emulsifier. This local emulsification may be the result of body temperature causing the wax particles to melt or it may be the result of pressure exerted during usage of the applicator, or it may be the result of both, the latter being usually the case In the instance of local emulsification by the effect of pressure, the emulsification process is driven by the (limited) pressure exerted by the user when applying the applicator, e.g. by rubbing it across the skin, dabbing it and the like. This causes the two phases to contact and form an emulsion locally.

In this local emulsification process, a limited amount of the phase without emulsifier is incorporated into the phase having the emulsifier. In a preferred embodiment, the aqueous phase of the dispersion contains a small amount of emulsifier, for example the emulsifier may be present in an amount from about 0.5 to about 5%, more in particular from about 1 to about 3%. In that instance some of the wax phase in the wax particles , of the dispersion is locally emulsified into the aqueous phase.

Although in preferred executions the wax dispersion is not present on the whole surface of the applicator, good release of the components contained in the wax dispersion is attained, in particular when the local emulsification process comes into play. Optimal release of active ingredients can also be achieved by making use of both above possibilities.

The products according to the invention can be for baby or adult use in a wide range of applications as personal care products, comprising, for example, baby cleansing, face or body cleansing, skin treatment or skin conditioning such as for example skin moisturization or treatment of the effects of skin aging, insect repellence, powder applicators, applicator for use as anti-perspirants, peeling, after-sun treatment, sunscreen, feminine hygiene, nappy rash, the latter preferably containing zinc oxide as active ingredient, and the like.

The products of the invention mainly are aimed for use as end products. In this instance the consumer is instructed to treat these products with water or with an aqueous lotion, which for example could be sold separately. Examples of products with low water content are the so-called 'dry applicators', which are aimed for use on a wet skin. Examples of this type of applicators are used in the shower or after bathing. Such dry applicators may also be recommended for use after wetting the product itself, e,g. with water or with an aqueous lotion that is provided separately.

Examples of products with relatively higher water content, e.g. from about 5 % to about 10 % are so-called 'intermediate dry applicators'.

The products of the invention may also find use as cleansing tools. They have been found to be more effective cleansers compared to products that have only an aqueous phase. This is due, i.a., by the fact that they are effective in removing both aqueous and lipid soils.

The products of the invention can be used as applicators of active substances or other ingredients having skin-beneficial properties (i.e. the so-called 'leave-on' products), in particular of the active substances mentioned herein, or they find use as combined cleanser and applicator of active substances in one product.

A still further advantage of those products where the wax dispersion is at the surface lies in the fact that they allow an improved transfer of actives onto the skin due to the presence of useful ingredients at the surface of the applicator. These offers advantages over products where the useful ingredients are in the inner phase of a typical o/w-emulsion.

The products according to the invention possess the additional advantage of being almost odorless (unless fragrances are added), environmentally friendly and biologically decomposable.

In view of these beneficial properties, the products of this invention can be used in a wide variety of cosmetic and personal care applications, but also in other cleaning or cleansing applications such as cleaning of hard surfaces (household care).

### Examples

### Examples 1-11

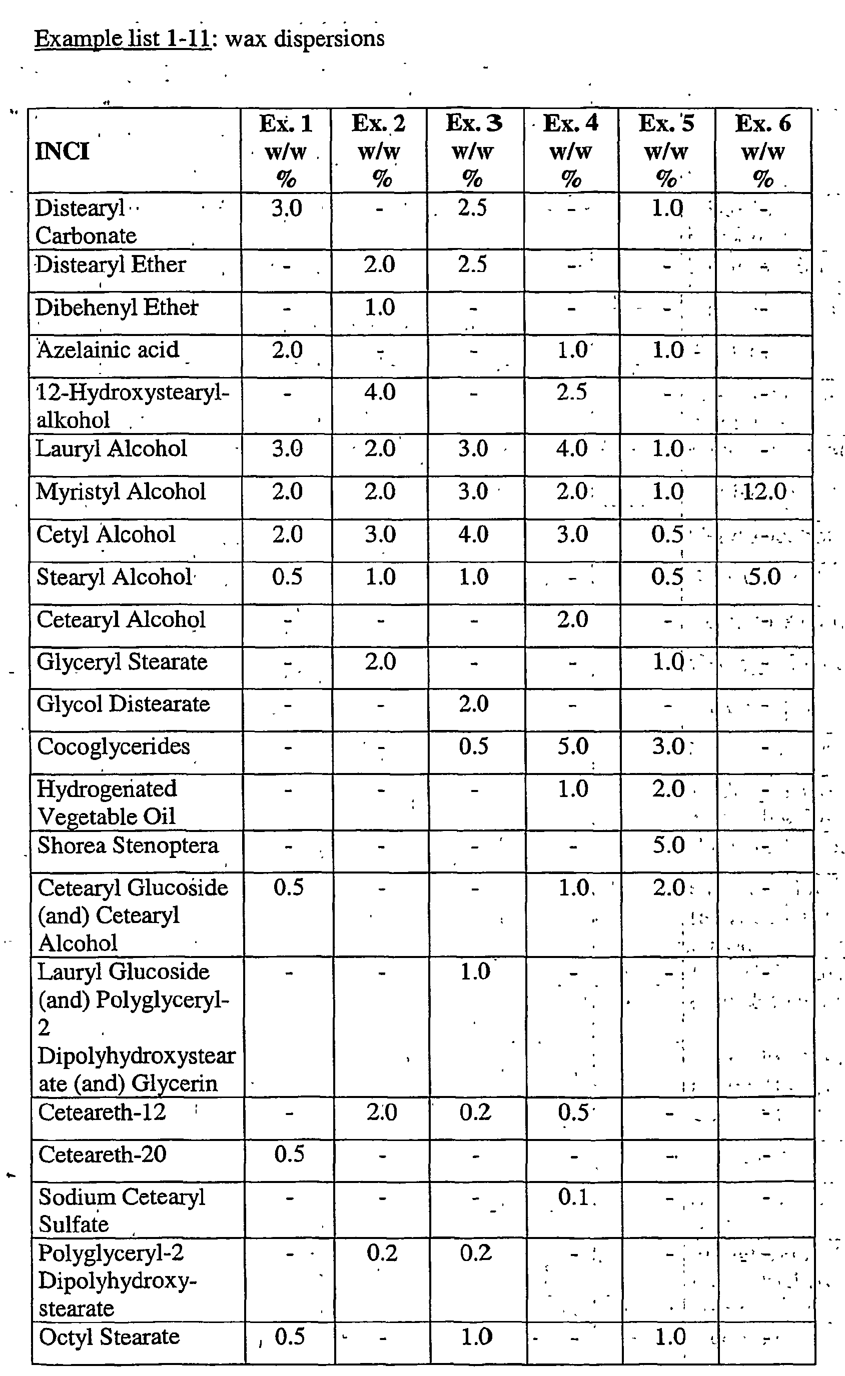

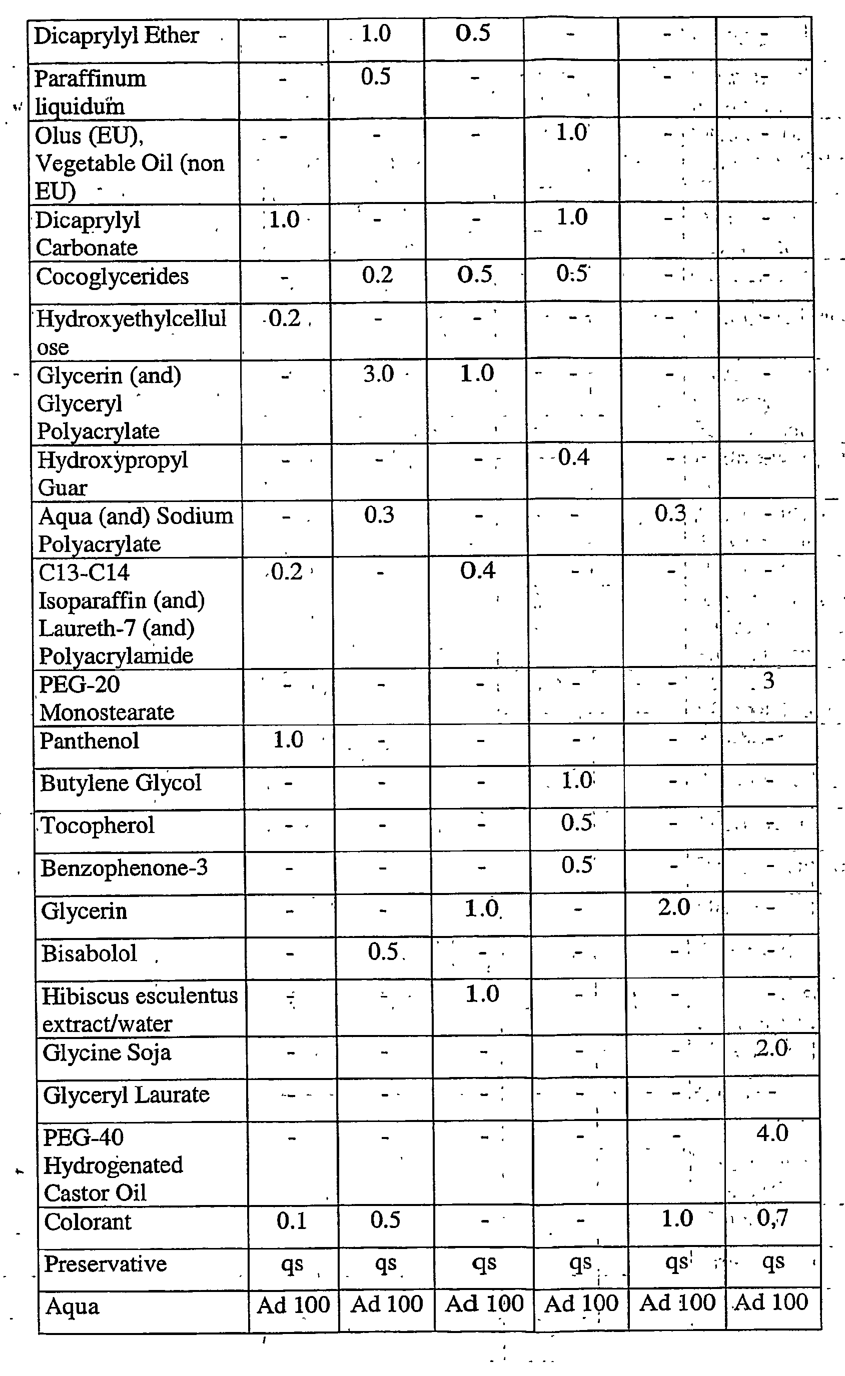

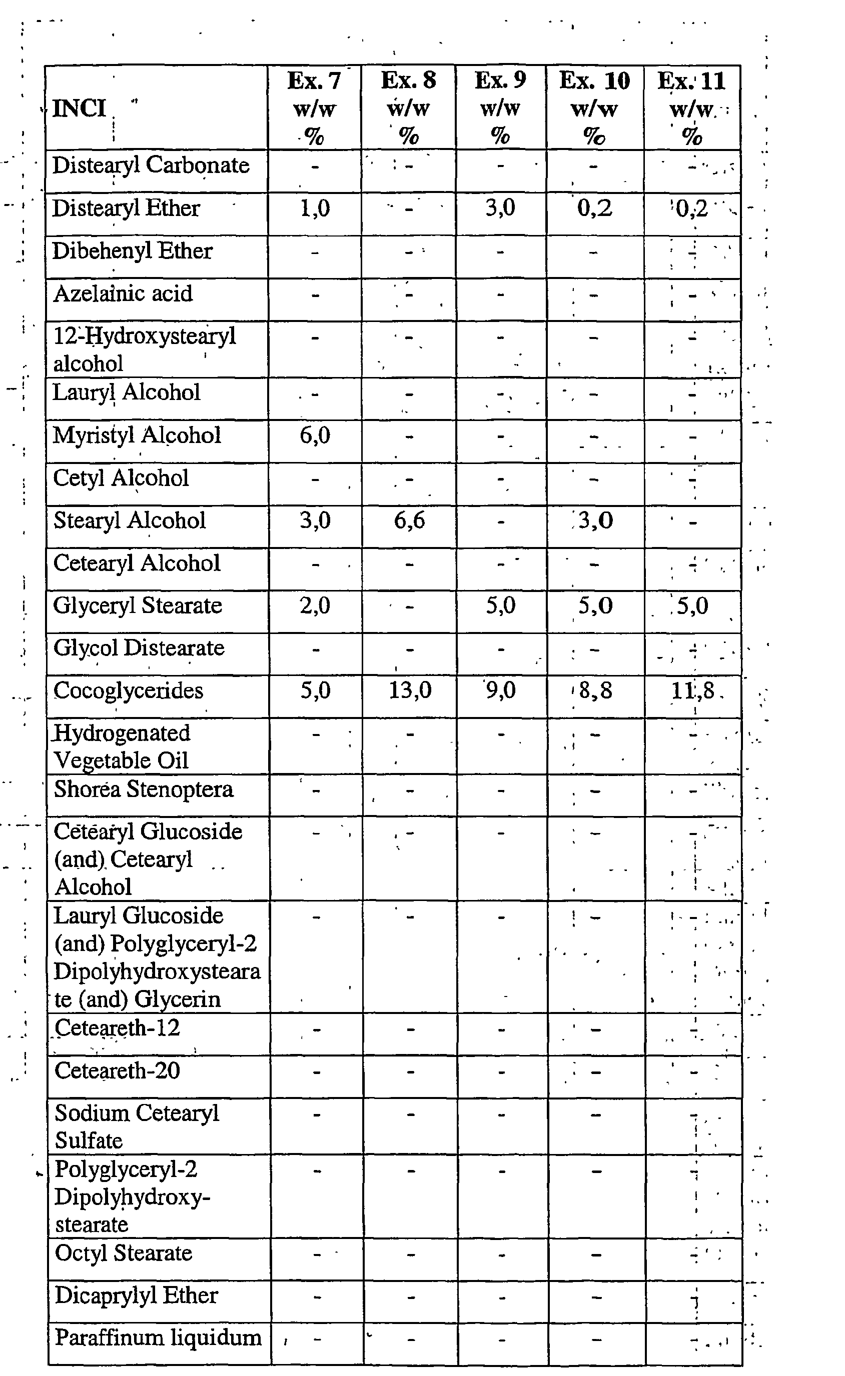

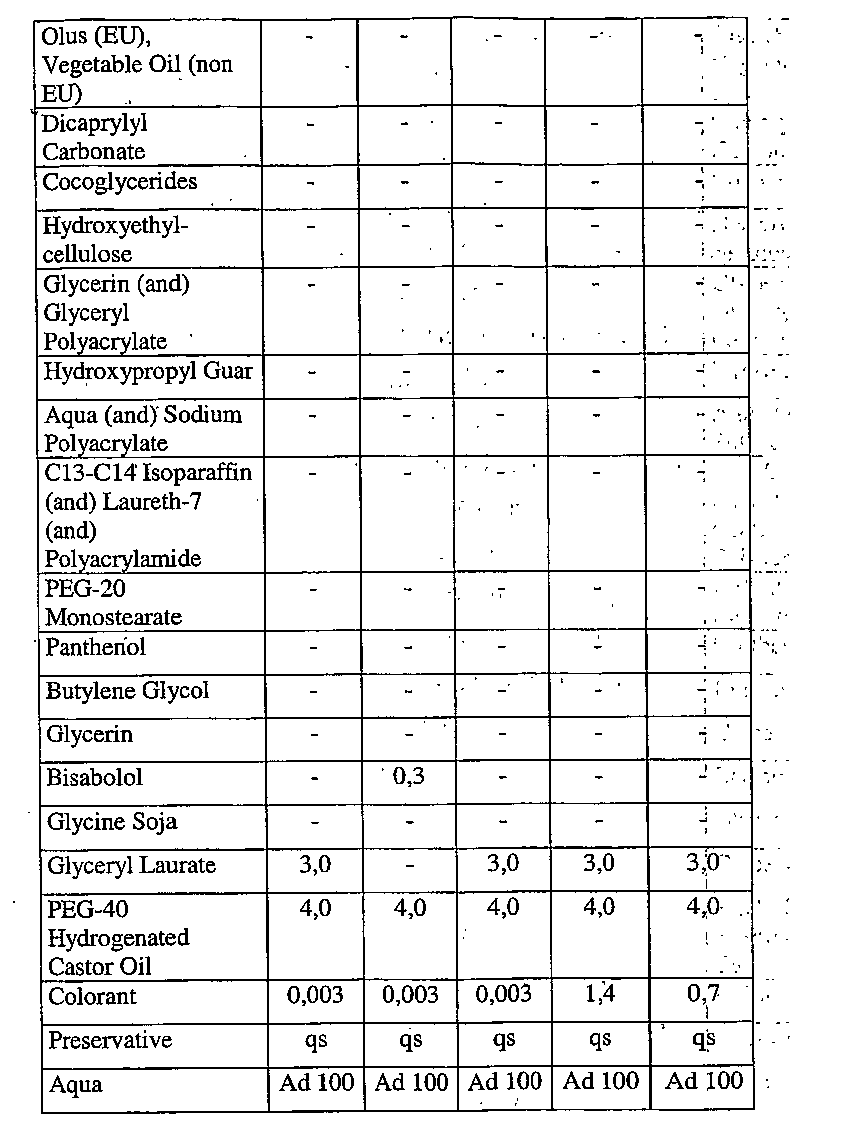

### Example list 12: aqueous phases

### Phase 12-A

- Aqua: 96.336 %
- Polysorbate 20: 0.600 %
- PEG-75 Lanolin: 0.100 %
- Parfum: 0.150%
- PEG-40 Hydrogenated Castor Oil: 0.400 %
- Propylene Glycol: 1.120 %
- Phenoxyethanol: 0.800%
- Tetrasodium EDTA: 0.078 %
- Chamornilla Recutita: 0.070%
- Ethoxydiglycol: 0.171 %
- Butylene Glycol: 0.035 %
- Glucose: 0.016%
- Iodopropynyl Butylcarbamate: 0.010%
- PEG- 4 Laurate: 0.090 %
- Citric Acid: 0.020%

### Phase 12-B

- Aqua: 98.252 %
- Phenoxyethanol: 0.800 %
- Iodopropynyl Butylcarbamate: 0.010 %
- PEG- 4 Laurate: 0.090%
- Parfum: 0.150 %
- Tetrasodium EDTA: 0.078%
- Citric Acid: 0.020 %
- Polysorbate 20: 0.600 %

### Phase 12-C

- Aqua: 97.250 %
- Glycerins: 1.000 %
- Phenoxyethanol: 0.800 %
- Iodopropynyl Butylcarbamate: 0.010 %
- PEG- 4 Laurate: 0.090 %
- Parfum: 0.150 %
- Tetrasodium EDTA: 0.078 %
- Citric Acid: 0.020 %
- Polysorbate 20: 0.600 %

### Phase 12-D

- Aqua: 96.332 %
- Glycerines: 1.000 %
- Phenoxyethanol: 0.800 %
- Polysorbate 20: 0.600 %
- PPG-15 Stearyl Ether: 0.400 %
- PEG-7 Glyceryl Cocoate: 0.100 %
- Propylene Glycol: 0.350 %
- Iodopropynyl Butylcarbamate: 0.010 %
- PEG- 4 Laurate: 0.090 %
- Chamomilla Recutita: 0.070 %
- Parfum: 0.150 %
- Tetrasodium EDTA: 0.078 %
- Citric Acid: 0.020 %

### Phase 12-E

- Aqua: 97.33 %
- Phenoxyethanol: 0.800 %
- Polysorbate 20: 0.600 %
- Sorbeth-30: 0.400 %
- Propylene Glycol: 0.350 %
- Dimethicone Copolyol: 0.100 %
- Iodopropynyl Butylcarbamate: 0.010 %
- PEG- 4 Laurate: 0.090 %
- Chamomilla Recutita: 0.070 %
- Parfum: 0.150 %
- Tetrasodium EDTA: 0.078 %
- Citric Acid: 0.020 %

### Phase 12-F

- Aqua: 97.332 %
- Phenoxyethanol: 0.800 %
- PEG-80 Sorbitan Laurate: 0.600 %
- Propylene Glycol: 0.350 %
- Sorbeth-30: 0.400 %
- Octyldecanol: 0.100 %
- Iodopropynyl Butylcarbamate: 0.010 %
- PEG-4 Laurate: 0.090 %
- Chamomilla Recutita: 0.070 %
- Parfum: 0.150 %
- Tetrasodium EDTA: 0.078 %
- Citric Acid: 0.020 %

### Phase 12-G

- Aqua: 97.332 %
- Phenoxyethanol: 0.800 %
- Polysorbate-20: 0.600 %
- PGG-15 Stearyl Ether: 0.400 %
- Propylene Glycol: 0.350 %
- Decyl Oleate: 0.100 %
- Iodopropynyl Butylcarbamate: 0.010 %
- PEG-4 Laurate: 0.090 %
- Chamomilla Recutita: 0.070 %
- Parfum: 0.150 %
- Tetrasodium EDTA: 0.078 %
- Citric Acid: 0.020 %

### Phase 12-H

- Sodium Myreth Sulfate: 10.00 %
- Lauryl Glucoside: 15.00 %
- Cocamidopropyl Betaine: 10.00 %
- Aqua: 64.50 %
- Parfum: 0.50 %

### Phase 12-I

- Sodium Laureth Sulfate: 20.00 %
- Decyl Glucoside: 5.00 %
- Cocamidopropyl Betaine: 8.00 %
- Laureth-2: 2.50 %
- Polysorbate-20: 1.00 %
- Aqua: 63.00 %
- Parfum: 0.50 %

### Phase 12-J

- Sodium Myreth Sulfate: 15.00 %
- Lauryl Glucoside: 10.00 %
- Laureth-2: 1.50 %
- Aqua: 73.00 %
- Parfum: 0.50 %

### Phase 12-K

- Emulgade® CM: 20.00 %
- Polysorbate 20: ' 0.80 %
- Coco-Glucoside: 2.50 %
- Phenoxyethanol: 1.00 %
- Cetylpyridinium Cloride: 0.10 %
- Tetrasodium EDTA: 0.20 %
- Aqua: 75.22 %
- Citric Acid: 0.08 %
- Parfum: 0.10 %

### Phase 12-L

- Emulgade^{®} SE-PF: 1.66 %
- Ceteareth-12: 0.94 %
- Lamesoft^{®} PO 65: 0.25 %
- Paraffinum Liquidum: 3.00 %
- Cetylpyridinium Cloride: 0.05 %
- Polysorbate-20: 1.00 %
- Citric Acid: 0.03 %
- Tetrasodium EDTA: 0.20 %
- Nipaguard® IPF: 0.10 %
- Aqua: 92.06 %
- C.I. 47005: 0.06%
- Parfum: 0.11 %

### Phase 12-M

- Propylene Glycol: 0.98 %
- PEG-40 Hydrogenated Castor Oil: 0.4 %
- Butylparaben: 0.075 %
- Methylparaben: 0.155 %
- Propylparaben: 0.1 %
- Phenoxyethanol: 0.7 %
- Polysorbate-20: 3.25 %
- Citric Acid .: 0.05 %
- Tetrasodium EDTA: 0.2 %
- Nipaguard® IPF: 0.098, %
- Aqua: 93.94 %
- Parfum: 0.15 %

### Example 13

Dry sponge consisting of two parts made of different material are glued together. One part is made of liquid cellulose. After drying, the sponge material forms a layer with the thickness of 37 mm. The sponge has a surface weight of 70 g/m2 and was impregnated with 10 g/m2 of an aqueous phase, which is prepared as set forth in example list 12. After that the material is cut into blocks of 135 x 90 x 37 mm. The other part of the product is made of polyurethane with the measures 135 x 90 x 5 mm. After gluing both parts together a wax dispersion as described in example list 1-11 is applied with 5 g/article onto the polyurethane side. The product is wrapped into single packs to lock the moisture in.

### Example 14

Dry sponge is made of liquid cellulose and cut after forming into blocks of 135 x 90 x 37 mm. The cellulose material was immersed into a wax dispersion as described in example list 1-11, which is released after wetting and while squeezing the sponge during use.

### Example 15

Dry sponge is made and treated with a wax dispersion as described in example 14. Subsequently the product is dried by blowing dry air.

### Example 16

The product manufactured as described in the previous example is sprayed with an aqueous formulation as described in example list 12. Subsequently the thus manufactured products are packed into a film packaging.

## Claims

1. A product comprising an applicator selected from the group consisting of a puff (pouf), pad, sponge, foam, glove, mitt, brush, swab, cotton ball and a bar, which comprises an applicator, other than a porous or absorbent sheet, said applicator has been contacted with a wax dispersion which comprises
(a) 1 - 75 w/w-% of a wax phase having a melting point higher than 25°C, that contains at least one oil or wax component selected from dialkyl(ene) ethers, dialkyl(ene) carbonates, dicarbonic acids and hydroxyfatty alcohols, and any mixture of these components, wherein the total amount of the dialkyl(ene) ethers, dialkyl(ene) carbonates, dicarbonic acids and the hydroxyfatty alcohols present in the wax phase, relative to the total weight amount of the wax phase, is in the range of 0.1 to 30 w/w-%, and wherein the wax particles have an average particle size, which is in the range of 0.5 to 100 µm, in particular in the range of 1 to 50 µm, more in particular from 1 to 50 µm, and
(b) 25 - 99 w/w-%, relative to the total weight of the dispersion, of an aqueous phase.

2. The product according to claim 1 wherein the wax dispersion additionally contains 0.01 - 5 % (w/w relative to the total weight of the dispersion) of a polymer.

3. The product according to claim 2 wherein the polymer is selected from the group of polyacrylates, polysaccharides, polyacrylamides or any mixture of these polymers.

4. The product according to any of the preceding claims wherein the wax phase comprises components selected from fats, waxes, fatty alcohols, fatty acids, and any mixture of these components.

5. The product according to any of the preceding claims wherein the wax dispersion further contains a suitable emulsifier.

6. The product according to claim 5 wherein the wax dispersion comprises:
(a) 1 - 50 weight % of a wax phase comprising:
(a1) 0.1 - 30 weight % of at least an oil or wax component selected from C₁₄-C₃₀-dialkyl ethers, C₁₄-C₃₀-dialkyl carbonates, C₄-C₃₄-dicarbonic acids or C₁₂-C₃₀-hydroxyfatty alcohols or any mixture thereof;
(a2) 0.1 - 10 % (w/w) of at least one oil;
(a3) 0.1 - 10 % (w/w) of at least one non-ionic emulsifier
(a4) 0.1 - 40 % (w/w) of at least one further waxy lipid component; w/w relative to the total weight of the wax dispersion;
(b) 50 - 99 % (w/w) of an aqueous phase; w/w relative to the total weight of the wax dispersion.

7. The product according to any of the preceding claims wherein the wax dispersion contains 5 - 30 weight-% of a wax phase, in particular 10 - 25 weight-% of a wax phase, relative to the total weight of the wax dispersion.

8. The product according to any of the preceding claims wherein the wax phase comprises mono-, di- or triglycerides, fatty alcohols, fatty acids or any combination of these ingredients.

9. The product according to any of the preceding claims wherein the additional aqueous phase or the wax dispersion, or both, contains one or more active substances.

10. The product according to claim 9 wherein the active substance(s) is or are antimicrobials, e.g. anti-bacterials and antifungals, anti-inflammatory agents, anti-irritating, anti-itching, anti-perspirant, anti-ageing, ant-stinging, soothing, calming agents.

11. The product according to any of the preceding claims wherein the applicator has been treated with an additional aqueous phase.

12. The product according to claim 11 wherein the applicator has been treated with the additional aqueous phase, after which the product has been dried, and to which subsequently the wax dispersion has been applied.

13. The product according to claim 11 wherein the applicator has been treated simultaneously with the additional aqueous phase and the wax dispersion, after which the product has been dried.

14. The product according to claim 11 wherein the applicator has been treated with the additional aqueous phase and to which subsequently the wax dispersion has been applied, whereafter the product has been dried.

15. The product according to claim 11 wherein the applicator has been contacted with the wax dispersion and has been subsequently treated with the additional aqueous phase, whereafter the product has been dried.

16. A method of manufacturing a product as claimed in any of the preceding claims, said method comprising contacting the applicator with the wax dispersion.

17. The method according to claim 16, said method further comprising treating the applicator with the additional aqueous phase either subsequently or simultaneously to the contacting with the wax dispersion.

18. The method of claim 16 or 17, said method further comprises a drying step.

19. The method of one of claims 16 to 18 wherein the additional aqueous phase is applied by spraying, dripping, immersing or running through a bath, and the wax phase is applied by spraying, contacting, printing or a direct contact process where there is a direct contact between the applicator and an application head having slit nozzles.

20. Use of a product as claimed in any of claims 1 to 15 as a cleanser.

## Patentansprüche

1. Produkt, umfassend einen Applikator, ausgewählt aus der Gruppe, bestehend aus einer Quaste [Puff], einem Pad, einem Schwamm, einem Schaumstoff, einem Handschuh, einem Fausthandschuh, einer Bürste, einem Tupfer, einem Wattebällchen und einem Stiel, der einen anderen Applikator als eine poröse oder absorbierende Lage umfasst, wobei der Applikator mit einer Wachsdispersion in Kontakt gebracht worden ist, die umfasst
(a) 1 - 75 w/w-% einer Wachsphase mit einem Schmelzpunkt höher als 25 °C, die zumindest eine Öl- oder Wachskomponente enthält, ausgewählt aus Dialkyl(en)-Ethern, Dialkyl(en)-Carbonaten, Dicarbonsäuren und Hydroxyfettalkoholen und einem Gemisch dieser Komponenten, wobei sich die Gesamtmenge der Dialkyl(en)-Ether, Dialkyl(en)-Carbonate, Dicarbonsäuren und Hydroxyfettalkohole, die in der Wachsphase vorliegen, relativ zur Gesamtgewichtsmenge der Wachsphase, im Bereich von 0,1 bis 30 w/w-% befindet, und wobei die Wachsteilchen eine durchschnittliche Teilchengröße im Bereich von 0,5 bis 100 µm, im Besonderen im Bereich von 1 bis 50 µm und noch spezieller im Bereich von 1 bis 50 µm aufweisen und
(b) 25 bis 99 w/w-%, relativ zum Gesamtgewicht der Dispersion, einer wässrigen Phase.

2. Produkt nach Anspruch 1, **dadurch gekennzeichnet, dass**
die Wachsdispersion zusätzlich 0,01 bis 5 % (w/w relativ zum Gesamtgewicht der Dispersion) eines Polymers enthält.

3. Produkt nach Anspruch 2, **dadurch gekennzeichnet, dass** das Polymer ausgewählt wird aus der Gruppe von Polyacrylaten, Polysacchariden, Polyacrylamiden oder einem Gemisch dieser Polymere.

4. Produkt nach einem der vorangehenden Ansprüche, **dadurch gekennzeichnet, dass** die Wachsphase Komponenten enthält, ausgewählt aus Fetten, Wachsen, Fettalkoholen, Fettsäuren und einem Gemisch dieser Komponenten.

5. Produkt nach einem der vorangehenden Ansprüche, **dadurch gekennzeichnet, dass** die Wachsdispersion weiterhin einen geeigneten Emulgator enthält.

6. Produkt nach Anspruch 5, **dadurch gekennzeichnet, dass** das die Wachsdispersion umfasst:
(a) 1 - 50 Gewichtsprozent einer Wachsphase, umfassend:
(a1) 0,1 - 30 Gewichtsprozent von zumindest einer Öl- oder Wachskomponente, ausgewählt aus C₁₄-C₃₀-Dialkylethern, C₁₄-C₃₀-Dialkylcarbonaten, C₄-C₃₄-Dicarbonsäuren oder C₁₂-C₃₀ Hydroxyfettalkoholen oder einem Gemisch derselben;
(a2) 0,1 - 10 % (w/w) von zumindest einem Öl;
(a3) 0,1 - 10 % (w/w) von zumindest einem nichtionogenen Emulgator;
(a4) 0,1 - 40 % (w/w) von zumindest einer weiteren Wachslipid-Komponente; w/w relativ zum Gesamtgewicht der Wachsdispersion;
(b) 50 - 99 % (w/w) einer wässrigen Phase; w/w relativ zum Gesamtgewicht der Wachsdispersion.

7. Produkt nach einem der vorangehenden Ansprüche, **dadurch gekennzeichnet, dass** die Wachsdispersion 5 - 30 Gewichtsprozent einer Wachsphase enthält, im Besonderen 10 - 25 Gewichtsprozent einer Wachsphase, relativ zum Gesamtgewicht der Wachsdispersion.

8. Produkt nach einem der vorangehenden Ansprüche, **dadurch gekennzeichnet, dass** die Wachsphase Mono-, Di- oder Triglyceride, Fettalkohole, Fettsäuren oder eine Kombination dieser Bestandteile umfasst.

9. Produkt nach einem der vorangehenden Ansprüche, **dadurch gekennzeichnet, dass** die zusätzliche wässrige Phase oder die Wachsdispersion, oder beide, einen oder mehr Wirkstoffe enthalten.

10. Produkt nach Anspruch 9, **dadurch gekennzeichnet, dass** der Wirkstoff/die Wirkstoffe ein Antimikrobiotikum ist / Antimikrobiotika sind, zum Beispiel Bakterizide und Fungizide, entzündungshemmende Mittel, reizungshemmende Mittel, juckreizhemmende Mittel, perspirationshemmende Mittel, Anti-Aging-Mittel, Mittel gegen Stiche, lindernde und beruhigende Mittel.

11. Produkt nach einem der vorangehenden Ansprüche, **dadurch gekennzeichnet, dass** der Applikator mit einer zusätzlichen wässrigen Phase behandelt worden ist.

12. Produkt nach Anspruch 11, **dadurch gekennzeichnet, dass** der Applikator mit der zusätzlichen wässrigen Phase behandelt worden ist, nach welcher das Produkt getrocknet worden ist, und auf welches nachfolgend die Wachsdispersion aufgebracht worden ist.

13. Produkt nach Anspruch 11, **dadurch gekennzeichnet, dass**
der Applikator gleichzeitig mit der wässrigen Phase und der Wachsdispersion behandelt worden ist, wonach das Produkt getrocknet wurde.

14. Produkt nach Anspruch 11, **dadurch gekennzeichnet, dass** der Applikator mit der zusätzlichen wässrigen Phase behandelt worden ist und auf welchen nachfolgend die Wachsdispersion aufgebracht worden ist, wonach das Produkt getrocknet wurde.

15. Produkt nach Anspruch 11, **dadurch gekennzeichnet, dass**
der Applikator in Kontakt mit der Wachsdispersion gebracht worden ist und danach mit der zusätzlichen wässrigen Phase behandelt worden ist, wonach das Produkt getrocknet wurde.

16. Verfahren zur Herstellung eines Produkts, wie in einem der vorangehenden Ansprüche beansprucht, wobei das Verfahren das Inkontaktbringen des Applikators mit der Wachsdispersion umfasst.

17. Verfahren nach Anspruch 16, wobei das Verfahren weiterhin das Behandeln des Applikators mit der zusätzlichen wässrigen Phase umfasst, entweder nach dem Inkontaktbringen mit der Wachsdispersion oder gleichzeitig damit.

18. Verfahren nach Anspruch 16 oder 17, wobei das Verfahren weiterhin einen Trocknungsschritt umfasst.

19. Verfahren nach einem der Ansprüche 16 bis 18, **dadurch gekennzeichnet, dass** die zusätzliche wässrige Phase aufgebracht wird durch Sprühen, Auftropfen, Eintauchen oder Führen durch ein Bad, und die Wachsphase aufgebracht wird durch Sprühen, Inkontaktbringen, Aufdrucken oder ein direktes Kontaktverfahren, bei dem es einen direkten Kontakt zwischen dem Applikator und einem Aufbringkopf mit Schlitzdüsen gibt.

20. Verwendung eines Produkts, wie in einem der Ansprüche 1 bis 15 beansprucht, als ein Reinigungsmittel.

## Revendications

1. Produit comprenant un applicateur choisi dans le groupe constitué par une houppette (pouffe), un tampon, une éponge, une mousse, un gant, une moufle, une brosse, un coton-tige, un tampon de coton et une barre, qui comprend un applicateur autre qu'une feuille poreuse ou absorbante, ledit applicateur ayant été mis en contact avec une dispersion de cire comprenant
(a) 1 à 75 % en poids d'une phase de cire possédant un point de fusion supérieur à 25°C et contenant au moins un composant à base d'huile ou de cire choisi parmi les éthers de dialkyl(èn)e, les carbonates de dialkyl-(èn)e, les acides dicarboniques et les hydroxy-alcools gras et n'importe quel mélange de ces composants, où la quantité totale d'éthers de dialkyl(èn)e, de carbonates de dialkyl(èn)e, d'acides dicarboniques et d'hydroxy-alcools gras présents dans la phase de cire, par rapport à la quantité totale en poids de la phase de cire, se situe dans la plage allant de 0,1 à 30 % en poids, et où les particules de cire possèdent une taille moyenne de particule se trouvant dans la plage allant de 0,5 à 100 µm, en particulier dans la plage allant de 1 à 50 µm, plus particulièrement de 1 à 50 µm, et
(b) 25 à 99 % en poids par rapport au poids total de la dispersion, d'une phase aqueuse.

2. Produit selon la revendication 1, dans lequel la dispersion de cire contient en outre 0,01 à 5 % (en poids par rapport au poids total de la dispersion) d'un polymère.

3. Produit selon la revendication 2, dans lequel le polymère est choisi parmi le groupe des polyacrylates, des polysaccharides, des polyacrylamides ou de n'importe quel mélange de ces polymères.

4. Produit selon l'une quelconque des revendications précédentes, dans lequel la phase de cire comprend des composants choisis parmi des matières grasses, des cires, des alcools gras, des acides gras et n'importe quel mélange de ces composants.

5. Produit selon l'une quelconque des revendications précédentes, dans lequel la dispersion de cire contient en outre un émulsifiant approprié.

6. Produit selon la revendication 5, dans lequel la dispersion de cire comprend :
(a) 1 à 50 % en poids d'une phase de cire comprenant :
(a1) 0,1 à 30 % en poids d'au moins un composant à base d'huile ou de cire choisi parmi les éthers de dialkyle en C₁₄ à C₃₀, les carbonates de dialkyle en C₁₄ à C₃₀, les acides dicarboniques en C₄ à C₃₄ ou les hydroxy-alcools gras en C₁₂ à C₃₀ ou n'importe quel mélange de ces composants ;
(a2) 0,1 à 10 % (en poids) d'au moins une huile ;
(a3) 0,1 à 10 % (en poids) d'au moins un émulsifiant non ionique ;
(a4) 0,1 à 40 % (en poids) d'au moins un composant lipidique cireux supplémentaire ; en poids par rapport au poids total de la dispersion de cire ;
(b) 50 à 99 % (en poids) d'une phase aqueuse ; en poids par rapport au poids total de la dispersion de cire.

7. Produit selon l'une quelconque des revendications précédentes, dans lequel la dispersion de cire contient 5 à 30 % en poids d'une phase de cire, en particulier 10 à 25 % en poids d'une phase de cire, par rapport au poids total de la dispersion de cire.

8. Produit selon l'une quelconque des revendications précédentes, dans lequel la phase de cire comprend des mono-, di- ou tri-glycérides, des alcools gras, des acides gras ou n'importe quelle combinaison de ces ingrédients.

9. Produit selon l'une quelconque des revendications précédentes, dans lequel la phase aqueuse supplémentaire ou la dispersion de cire, ou les deux, contient/contiennent une ou plusieurs substances actives.

10. Produit selon la revendication 9, dans lequel la ou les substance(s) active(s) est/sont des agents antimicrobiens, par exemple des agents antibactériens et des agents antifongiques, des agents anti-inflammatoires, des agents anti-irritation, des agents anti-démangeaison, des agents anti-transpirants, des agents anti-vieillissement, des agents anti-urticants, des agents apaisants, des agents calmants.

11. Produit selon l'une quelconque des revendications précédentes, dans lequel l'applicateur a été traité avec une phase aqueuse supplémentaire.

12. Produit selon la revendication 11, dans lequel l'applicateur a été traité avec la phase aqueuse supplémentaire, après quoi le produit a été séché, puis la dispersion de cire a été appliquée sur celui-ci.

13. Produit selon la revendication 11, dans lequel l'applicateur a été traité simultanément avec la phase aqueuse supplémentaire et la dispersion de cire, après quoi le produit a été séché.

14. Produit selon la revendication 11, dans lequel l'applicateur a été traité avec la phase aqueuse supplémentaire puis la dispersion de cire a été appliquée sur celui-ci, après quoi le produit a été séché.

15. Produit selon la revendication 11, dans lequel l'applicateur a été mis en contact avec la dispersion de cire puis traité avec la phase aqueuse supplémentaire, après quoi le produit a été séché.

16. Méthode de fabrication d'un produit selon l'une quelconque des revendications précédentes, ladite méthode comprenant la mise en contact de l'applicateur avec la dispersion de cire.

17. Méthode selon la revendication 16, ladite méthode comprenant en outre le traitement de l'applicateur avec la phase aqueuse supplémentaire soit en même temps, soit après le contact avec la dispersion de cire.

18. Méthode selon la revendication 16 ou 17, ladite méthode comprenant en outre une étape de séchage.

19. Méthode selon l'une quelconque des revendications 16 à 18, dans laquelle la phase aqueuse supplémentaire est appliquée par pulvérisation, égouttage, immersion ou passage dans un bain, et la phase de cire est appliquée par pulvérisation, contact, impression ou par un procédé de contact direct où l'applicateur est en contact direct avec une tête d'application possédant des buses à fente.

20. Utilisation d'un produit selon l'une quelconque des revendications 1 à 15 comme nettoyant.
